# EUROPEAN PATENT SPECIFICATION

(11) **EP 0 418 591 B1**
(45) Date of publication and mention of the grant of the patent: **21.06.1995**
(21) Application number: 90116248.7
(22) Date of filing: 24.08.1990
(51) Int. Cl.: C07D 489/12, A61K 31/485

(54) **(+)-Isomers of endoetheno/endoethano-epoxymorphinan derivatives as antitussive agents**
(+)-Isomere von Endoetheno/Endoethano-epoxymorphinan-Derivaten als Mittel gegen Husten
(+)-Isomères des dérivés d'endoéthéno/endoéthano-époxymorphinane comme agents antitussifs

(30) Priority: 24.08.1989 US 398213; 20.08.1990 US 568732
(43) Date of publication of application: 27.03.1991
(73) Proprietor: G.D. Searle & Co., Chicago Illinois 60680 (US); NATIONAL INSTITUTES OF HEALTH, Bethesda, Maryland 20892 (US)
(72) Inventor: Rice, Kenner C., Bethesda, Maryland 20817 (US); Farah, John M., Jr., St. Louis, Missouri 63104 (US); Grayson, Neile A., Gaithersburg, Maryland 20878 (US)
(74) Representative: Wolff, Hans Joachim, Dr.jur. Dipl.-Chem.

(56) References cited:
- EP-A- 0 270 290
- DE-A- 2 022 899
- GB-A- 1 178 537
- US-A- 3 474 101
- US-A- 4 521 601
- NATURAL PRODUCTS CHEMISTRY 1984, 1985, Elsevier Science Publ.B.V.,Amsterdampages 15 - 24; A. BROSSI et al.: "Efficient Syntheses and biological evaluation of novel [+]-morphinans"
- NATIONAL INSTITUTE ON DRUG ABUSE - RESEARCH MONOGRAPH SERIES, vol. 49, March 1984, Rockville, Maryland, US; pages 77 - 84; T.T. CHAU et al.: "Antitussive Potencies of l- and d-Opiates and Their Inhibition of Codeine Binding"
- SCIENCE, vol. 198, no. 4319, 25 November 1977, pages 842 - 845; Y.F. JACQUET etal.: "Stereospecific and Nonstereospecific Effects of [+]- and [-]-Morphine:Evidence for a New Class of Receptors?"
- JOURNAL OF PHARMACOLOGY AND EXPERIMENTAL THERAPEUTICS, vol. 215, no. 3, 1980,pages 668 - 672; T.T. CHAU et al.: "Comparative Studies of the Pharmacological Effects of the d- and l-Isomers of Codeine"
- JOURNAL OF PHARMACOLOGY AND EXPERIMENTAL THERAPEUTICS, vol. 227, no. 3, 1983,pages 723 - 731; S. HERLING et al.: "Discriminative Stimulus Effects of Dextrorphan in Pigeons"

## Description

### Field of the Invention

This invention relates to antitussive agents for suppression of cough. Of particular interest is a class of (+)-enantiomers of epoxymorphinan derivatives which possess antitussive potency but which lack narcotic side effects.

### Background of the Invention

Coughing is a reflex action which is a symptom of many diseases. Coughing is desirable and essential for clearing the airways in numerous clinical situations. In many circumstances, however, suppression of cough by treatment with antitussive drugs is indicated, since persistent cough can irritate the respiratory system and weaken the patient. Persistent cough can thus exacerbate existing disease conditions or cause further complications.

There are many known cough-suppression agents. For example, certain opiates such as morphine-like derivatives including, in particular, (-)-codeine, can act on the central nervous system to suppress the cough reflex. Cough suppression by opiates and by benzomorphinans such as dextromethorphan is thought to be mediated within the central nervous system [N.B. Eddy et al, World Health Organization: Geneva, Ch. 3, 127-156 (1970)]. Although never specifically identified as the site where centrally active antitussives operate, regulatory nuclei in the dorsal medulla process the afferent signals of tracheobronchial irritation that result in efferent stimulation of the cough reflex [H. L. Borison, Am. J. Physiol., 154, 55-62 (1948)].

The antitussive effects have been described for several (+)-isomers of certain natural opiate derivatives including (+)-morphine, (+)-dihydromorphinone and (+)-dihydrocodeine [T. Takebe, IN: K. Gato (ed.) Kitasato Institute, 115-122 (1964)], and also for (+)-codeine [T. T. Chau et al, J. Pharmacol. Exp. Ther., 215, 668-672 (1980)]. Other (+)-isomers of natural opiates, such as (+)-thebaine, (+)-oxymorphone and (+)-morphinan-6-one, have been found to have significant antitussive activity [A. Brossi et al, "Proceedings of the 14th International Symposium on the Chemistry of Natural Products", IUPAC, Poznan, Poland, Abst., p. 27 (1984)]. Also (+)-codeine and (+)-morphine have been studied in binding site assays believed to be predictive of antitussive activity [T. T. Chau et al, NIDA Research Monograph 49, 77-84 (1983)]. Alicyclic endoethenotetrahydrothebaines carboxylates having antitussive effects are described in GB-A-1178537.

Some unnatural [i.e., the (+)] isomers of opiates which have antitussive activity also have been shown to lack narcotic side effects typically associated with (-)-opiate isomers [T. Takebe, Id., supra]. For example, the unnatural isomer of morphine is not bound at opiate receptors in vitro [Y. F. Jacquet et al, Science, 198, 844-845 (1977)] and consequently (+)-morphine induces neither analgesia nor respiratory depression [T. Takebe, Id., supra]. Similarly, (+)-codeine does not bind at opiate receptors in vitro and consequently (+)-codeine is not analgesic in vivo [T. T. Chau et al, J. Pharmacol. Exp. Therap., 215, 668-672 (1980)]. In drug discrimination paradigms, the (+)-isomers of morphine and codeine were inactive as opiates or as psychotomimetics [S. Herling et al, J. Pharmacol. Exp. Therap. 227, 723-731 (1983)].

(-)-Thebaine derivatives, which are natural opiates, are known to have other CNS pharmacological properties. For example, U.S. Patent No. 3,474,101 to K. W. Bentley describes a series of 6,4-endoethenotetrahydrothebaine derivatives, including 6,14-endoetheno-7-α-(2-hydroxy-3-methyl-2-butyl)tetrahydrooripavine [also known as (-)-etorphine]. These compounds, especially (-)-etorphine, are asserted to possess potent analgesic properties. The compound (-)-etorphine has been investigated extensively for its pharmacological profile. It has been found, for example, that (-)-etorphine, while being a highly potent analgesic, also induces severe respiratory depression [G. F. Blane et al, Br. J. Pharmac. Chemother., 30, 11-22 (1967)]. Such a narcotic side-effect may be attributed to the stable, stereoselective binding of (-)-etorphine to opiate receptors [E. J. Simon et al, Proc. Nat. Acad. Sci. USA, 70, 7, 1947-1949 (1973)]. Studies of (-)-etorphine in man indicate that (-)-etorphine is a morphine-like drug with a high abuse potential [D. R. Jasinski et al, Clin. Pharmacol. Ther., 17, 3, 267-272 (1975)]. These narcotic side-effect properties render (-)-etorphine clinically unsuitable for any human therapeutic utility.

### BRIEF DESCRIPTION OF DRAWING

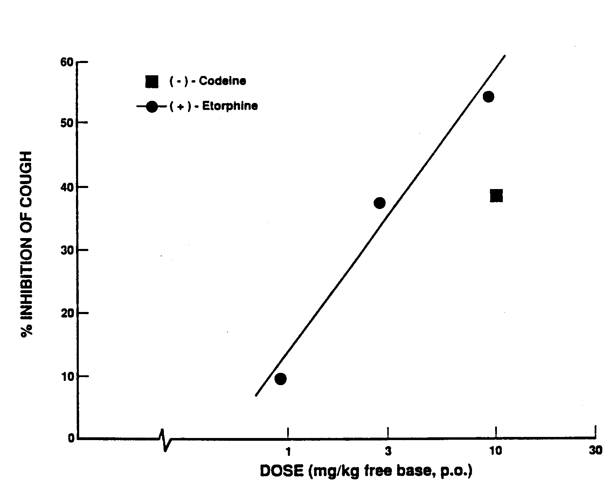

Fig. 1 is a graph showing suppression of cough in guinea pig by oral administration of the commercially-available compound (-)-codeine and by oral administration of (+)-etorphine which is a compound of the invention.

### DESCRIPTION OF THE INVENTION

Cough suppression in a subject susceptible to or experiencing cough may be effected by administering to such subject an antitussive-effective amount or cough-suppressing-effective amount of an endoetheno/endoethano-epoxymorphinan derivative of Formula I: 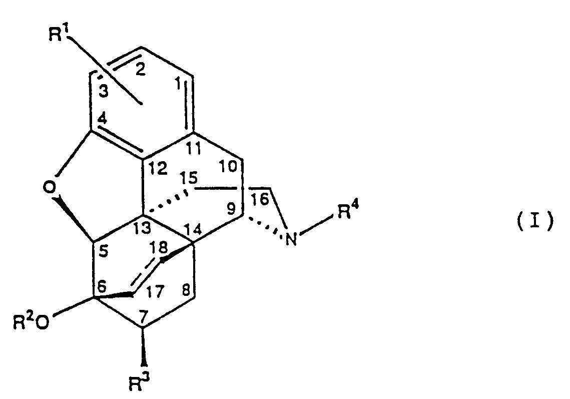
wherein R¹ is selected from hydrido, hydroxy, alkyl, hydroxyalkyl, haloalkyl, cycloalkyl, cycloalkylalkyl, alkoxyalkyl, alkoxy, alkoxycarbonyl, alkylcarboxyl, aryl, aralkyl, aryloxy, arylthio, alkylthio, amino, amido and carboxyl; wherein R² is selected from hydrido, alkyl, hydroxyalkyl, haloalkyl, alkoxyalkyl, haloacyl, aryl and aralkyl; wherein R³ is selected from alkyl, hydroxyalkyl, haloalkyl, cycloalkyl, cycloalkylalkyl, alkoxyalkyl, alkenylalkyl, alkoxy, aryl, aralkyl, aryloxy, alkylthio, amino, amido, carboxyl and 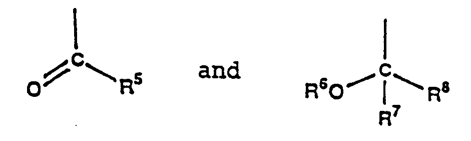
wherein each of R⁵, R⁶, R⁷ and R⁸ is independently selected from hydrido, alkyl, cycloalkyl, phenyl and phenalkyl; wherein R⁴ is selected from hydrido, alkyl, hydroxyalkyl, haloalkyl, cyano, cycloalkyl, cycloalkylalkyl, cycloalkylcarbonyl, alkenylalkyl, alkoxyalkyl, aryl, aralkyl, and carboxyl; wherein any of the foregoing R¹, R², R³ and R⁴ substituents having a substitutable position may be substituted with one or more groups selected from hydroxy, halo, alkyl, cycloalkyl, aryl and aralkyl; and wherein the dashed line between the 17- and 18-position carbon atoms of the carbon-carbon bridge connecting the 6-and 14-position carbon atoms represents a single bond or a double bond; or a pharmaceutically-acceptable salt thereof.

Compounds of Formula I are optically active detrorotatory [(+)] enantiomers. These (+)-isomers (i.e., the "unnatural" isomers) are expected to possess non-narcotic, antitussive properties, whereas the (-)isomer counterpart compounds would be expected to possess narcotic properties. Inasmuch as the nomenclature of the (-)-isomer compounds is fairly well established, the (+)-isomer compounds of this invention shall be named by reference to such (-)-isomer nomenclature. Thus, the compound 7α-(1-(R)-hydroxy-1-methylbutyl)-6,14-endo-ethenotetrahydrooripavine is also known as (-)-etorphine. A compound of the invention, for example, (+)-etorphine, may be named as the (+)-enantiomer of 7α-(1-(R)-hydroxy-1-methylbutyl)-6,14-endoethenotetrahydrooripavine.

A preferred class of compounds within Formula I consists of those compounds wherein R¹ is selected from hydrido, hydroxy, alkyl, hydroxyalkyl, alkoxyalkyl, alkoxy, alkoxycarbonyl, alkylcarboxyl, phenoxy and phenylthio; wherein R² is selected from hydrido, alkyl, hydroxyalkyl, alkoxyalkyl, phenyl and phenalkyl; wherein R³ is selected from alkyl, hydroxyalkyl, haloalkyl, cycloalkyl, cycloalkylalkyl, cycloalkylcarbonyl, alkenylalkyl, acyl, alkoxyalkyl and 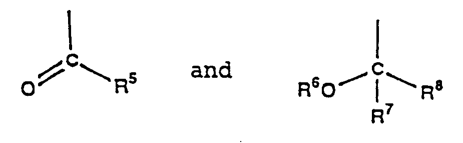
wherein each of R⁵, R⁶, R⁷ and R⁸ is independently selected from hydrido, alkyl,cycloalkyl, phenyl and phenalkyl;
wherein R⁴ is selected from hydrido, alkyl, hydroxyalkyl, haloalkyl, cyano, cycloalkyl, cycloalkylalkyl, cycloalkylcarbonyl, alkenylalkyl, alkoxyalkyl, phenyl and phenalkyl; and wherein any of the foregoing R¹, R², R³ and R⁴ substituents having a substitutable position may be substituted with one or more groups selected from hydroxy, halo, alkyl, cycloalkyl, phenyl and phenalkyl.

A more preferred class of compounds within Formula I consists of those compounds wherein R¹ is selected from hydroxy, alkyl, hydroxyalkyl, alkoxyalkyl, alkoxy, alkylcarboxyl, and phenoxy; wherein R² is selected from hydrido, alkyl, hydroxyalkyl, alkoxyalkyl, phenyl and phenalkyl; wherein R³ is selected from alkyl, hydroxyalkyl, haloalkyl, cycloalkyl, cycloalkylalkyl, alkoxyalkyl and 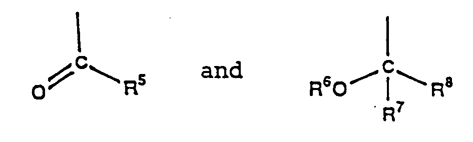
wherein each of R⁵, R⁶, R⁷ and R⁸ is independently selected from hydrido, alkyl, cycloalkyl, phenyl and phenalkyl;
wherein R⁴ is selected from hydrido, alkyl, hydroxyalkyl, cyano, cycloalkyl, cycloalkylalkyl, cycloalkylcarbonyl, allyl, alkoxyalkyl, phenyl and phenalkyl; and wherein any of the foregoing R¹, R², R³ and R⁴ substituents having a substitutable position may be substituted with one or more groups selected from hydroxy, halo, alkyl, cycloalkyl, phenyl and phenalkyl.

An even more preferred class of compounds within Formula I consists of those compounds wherein R¹ is selected from hydroxy, alkylcarboxyl, alkoxy and phenoxy; wherein R² is selected from hydrido, alkyl, and phenyl; wherein R³ is selected from 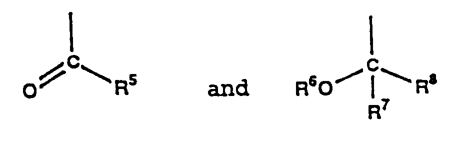
wherein each of R⁵, R⁶, R⁷ and R⁸ is independently selected from hydrido, alkyl, cycloalkyl, phenyl and phenalkyl; wherein R⁴ is selected from hydrido, alkyl, cyano, cycloalkyl, cycloalkylalkyl, cycloalkylcarbonyl, allyl, phenyl, benzyl, phenethyl and phenpropyl; and wherein any of the foregoing R¹, R², R³, R⁴, R⁵, R⁶, R⁷ and R⁸ substituents having a substitutable position may be substituted with one or more groups selected from hydroxy, halo, alkyl and cycloalkyl.

A still more preferred class of compounds within Formula I consists of those compounds wherein R¹ is selected from hydroxy, alkylcarboxyl, alkoxy and phenoxy; wherein R² is selected from hydrido, alkyl, acyl and phenyl; wherein each of R⁵, R⁶, R⁷ and R⁸ is independently selected from hydrido, alkyl and cycloalkyl; and wherein R⁴ is selected from hydrido, cyano, alkyl, cycloalkyl, cycloalkylalkyl, cycloalkylcarbonyl and allyl.

A highly preferred class of compounds within Formula I consists of those compounds wherein R¹ is selected from hydroxy, methylcarboxyl, methoxy, ethoxy, n-propoxy, isopropoxy, n-butoxy, isobutoxy, sec-butoxy, tert-butoxy and phenoxy; wherein R² is selected from hydrido, methyl, ethyl, n-propyl, isopropyl, n-butyl, isobutyl, sec-butyl, tert-butyl, acetyl, and phenyl; wherein each of R⁵, R⁶, R⁷ and R⁸ is independently selected from hydrido, methyl, ethyl, n-propyl, isopropyl, n-butyl, isobutyl, sec-butyl, tert-butyl; and wherein R⁴ is selected from hydrido, cyano, methyl, ethyl, n-propyl, isopropyl, n-butyl, isobutyl, sec-butyl, tert-butyl, cyclopropyl, methyl, allyl and cyclopropylcarbonyl.

A more highly preferred class of compounds within Formula I consists of those compounds wherein each of R⁵, R⁷, and R⁸ is selected from methyl, ethyl, n-propyl, isopropyl, n-butyl, isobutyl, sec-butyl tert-butyl; and wherein R⁶ is hydrido.

An even more highly preferred class of compounds within Formula I consists of those compounds wherein R³ is selected from

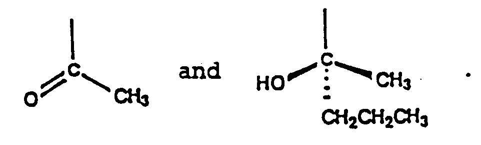

Compounds within this most highly preferred class may be divided into two sub-classes depending upon the presence of either a single bond or a double bond between the 17-position carbon and 18-position carbon of the carbon-carbon bridge connecting the 6-position carbon and 14-position carbon of Formula I.

A most highly preferred first sub-class of compounds within Formula I consists of those compounds wherein said compound has a double bond between the 17- and 18-position carbon atoms of the carbon-carbon bridge connecting the 6- and 14-position carbon atoms. Specific compounds within this first sub-class are the following: (+)-enantiomer of 7α-(1-(R)-hydroxy-1-methylbutyl)-6,14-endo-ethenotetrahydrooripavine [(+)-etorphine]; (+)-enantiomer of 7α-(1-(R)-hydroxy-1-methylbutyl)-6,14-endo-ethenotetrahydrothebaine; and (+)-enantiomer of 7α-acetyl-6,14-endo-ethenotetrahydrothebaine [(+)-thevinone].

A second sub-class of most highly preferred compounds consists of those compounds wherein said compound has a single bond between the 17- and 18-position carbon atoms of the carbon-carbon bridge connecting the 6- and 14-position carbon atoms. A group of specific compounds within this second sub-class consists of the following: (+)-enantiomer of 7α-acetyl-6,14-endo-ethanotetrahydrothebaine; (+)-enantiomer of 7α-(1-hydroxy-1-methylethyl)-6,14-endo-ethanotetrahydrothebaine; (+)-enantiomer of N-cyano-7α-(1-hydroxy-1-methylethyl)-6,14-endo-ethanotetrahydronorthebaine; (+)-enantiomer of 7α-(1-hydroxy-1-methylethyl)-6,14-endo-ethanotetrahydronororipavine; (+)-enantiomer of N-cyclopropylcarbonyl-7a-(1-hydroxy-1-methylethyl)-6,14-endo-ethanotetrahydronororipavine; and (+)-enantiomer of N-cyclopropylmethyl-7α-(1-hydroxy-1-methylethyl)-6,14-endo-ethanotetrahydronororipavine. Within this group, the (+)-enantiomer of N-cyclopropylmethyl-7α-(1-hydroxy-1-methylethyl)-6,14-endo-ethanotetrahydronororipavine [(+)-diprenorphine] is of highest interest.

Another group of specific compounds within this second sub-class consists of the following: (+)-enantiomer of 7α-(1-hydroxy-1-tert-butyl-ethyl)-6,14-endo-ethanotetrahydrothebaine; (+)-enantiomer of N-cyano-7α-(1-hydroxy-1-tert-butyl-ethyl)-6,14-endo-ethanotetrahydronorthebaine; and (+)-enantiomer of N-cyclopropylmethyl-7α-(1-hydroxy-1-tert-butyl-ethyl)-6,14-endo-ethanotetrahydronororipavine. Within this group, the (+)-enantiomer of N-cyclopropylmethyl-7α-(1-(R)-hydroxy-1-methyl-tert-butyl)-6,14-endo-ethanotetrahydronororipavine [(+)-buprenorphine] is of highest interest.

The term "hydrido" denotes a single hydrogen atom (H). This hydrido group may be attached, for example, to an oxygen atom to form a hydroxyl group, or, as another example, two hydrido groups may be attached to a carbon atom to form a -CH₂- group. Where the term "alkyl" is used, either alone or within other terms such as "haloalkyl", "aralkyl" and "hydroxyalkyl", the term "alkyl" embraces linear or branched radicals having one to about ten carbon atoms unless otherwise specifically described. Preferred alkyl radicals are "lower alkyl" radicals having one to about five carbon atoms. The term "cycloalkyl" embraces radicals having three to ten carbon atoms, such as cyclopropyl, cyclobutyl, cyclohexyl and cycloheptyl. The term "haloalkyl" embraces radicals wherein any one or more of the carbon atoms is substituted with one or more halo groups, preferably selected from bromo, chloro and fluoro. Specifically embraced by the term "haloalkyl" are monohaloalkyl, dihaloalkyl and polyhaloalkyl groups. A monohaloalkyl group, for example, may have either a bromo, a chloro, or a fluoro atom within the group. Dihaloalkyl and polyhaloalkyl groups may be substituted with two or more of the same halo groups, or may have a combination of different halo groups. Examples of a dihaloalkyl group are dibromomethyl, dichloromethyl and bromochloromethyl. Examples of a polyhaloalkyl are trifluoromethyl, 2,2,2-trifluoroethyl, perfluoroethyl and 2,2,3,3-tetrafluoropropyl groups. The term "alkoxy", embraces linear or branched oxy-containing radicals having an alkyl portion of one to about ten carbon atoms, such as methoxy, ethoxy, isopropoxy and butoxy. The term "alkylthio" embraces radicals containing a linear or branched alkyl group, of one to about ten carbon atoms attached to a divalent sulfur atom, such as a methythio group. The term "aryl" embraces aromatic radicals such as phenyl, naphthyl and biphenyl. Preferred aryl groups are those consisting of one, two, or three benzene rings. The term "aralkyl" embraces aryl-substituted alkyl radicals such as benzyl, diphenylmethyl, triphenylmethyl, phenylethyl, phenylbutyl and diphenylethyl. The terms "benzyl" and "phenylmethyl" are interchangeable. The terms "aryloxy" and "arylthio" denote radical respectively, aryl groups having an oxygen or sulfur atom through which the radical is attached to a nucleus, examples of which are phenoxy and phenylthio.

"Lower alkanoyl" is an example of a more preferred sub-class of acyl. The term "amido" denotes a radical consisting of nitrogen atom attached to a carbonyl group, which radical may be further substituted in the manner described herein. The amido radical can be attached to the nucleus of a compound of the invention through the carbonyl moiety or the nitrogen atom of the amido radical. The term "alkenylalkyl" denotes a radical having a double-bond unsaturation site between two carbons, and which radical may consist of only two carbons or may be further substituted with alkyl groups which may optionally contain additional double-bond unsaturation. For any of the foregoing defined radicals, preferred radicals are those containing between one and about ten carbon atoms.

Within the compounds of the invention of the invention described herein are the pharmaceutically-acceptable salts of such compounds including acid addition salts and base addition salts. The term "pharmaceutically-acceptable salts" embraces salts commonly used to form alkali metal salts and to form addition salts of free acids or free bases. The nature of the salt is not critical, provided that it is pharmaceutically-acceptable. Suitable pharmaceutically-acceptable acid addition salts of compounds of the invention may be prepared from an inorganic acid or from an organic acid. Examples of such inorganic acids are hydrochloric, hydrobromic, hydroiodic, nitric, carbonic, sulfuric and phosphoric acid. Appropriate organic acids may be selected from aliphatic, cycloaliphatic, aromatic, araliphatic, heterocyclic, carboxylic and sulfonic classes of organic acids, examples of which are formic, acetic, propionic, succinic, glycolic, gluconic, lactic, malic, tartaric, citric, ascorbic, glucuronic, maleic, fumaric, pyruvic, aspartic, glutamic, benzoic, anthranilic, p-hydroxybenzoic, salicyclic, phenylacetic, mandelic, embonic (pamoic), methansulfonic, ethanesulfonic, 2-hydroxyethanesulfonic, pantothenic, benzenesulfonic, toluenesulfonic, sulfanilic, mesylic, cyclohexylaminosulfonic, stearic, algenic, β-hydroxybutyric, malonic, galactaric and galacturonic acid. Suitable pharmaceutically-acceptable base addition salts of the compounds include metallic salts made from aluminium, calcium, lithium, magnesium, potassium, sodium and zinc or organic salts made from N,N′-dibenzylethylenediamine, chloroprocaine, choline, diethanolamine, ethylenediamine, meglumine (N-methylglucamine) and procaine. All of these salts may be prepared by conventional means from the corresponding conjugates described herein by reacting, for example, the appropriate acid or base with the compound.

### GENERAL SYNTHETIC PROCEDURES

The following general procedures show preparation of compounds within Formula I. 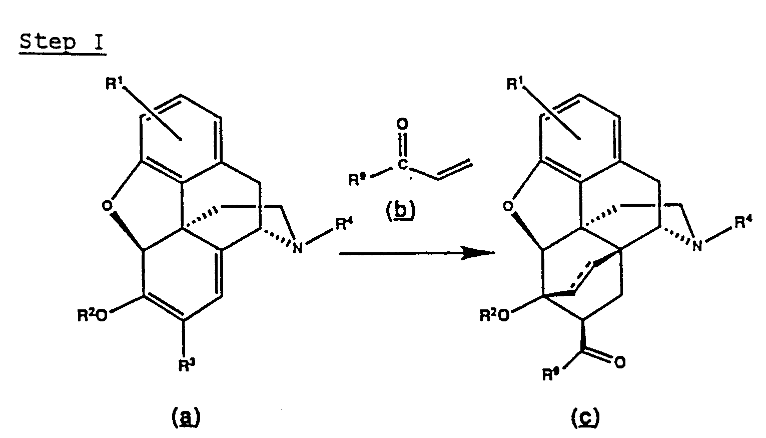
In Step I of the general procedure, an epoxymorphinan (+)-isomer (or (+)-thebaine) starting material (a) is converted to a (+)-thevinone-type intermediate (c). Conversion is effected by use of a substituted vinyl ketone (b) having a substituent R⁹ which is a precursor residue to be included within the R³ substituent of the end product formed in Step II and as shown in Formula I. Typically, R⁹ is hydrido or a linear or branched alkyl group, preferably lower alkyl such as methyl or ethyl, or aralkyl such as benzyl or phenethyl. The substituents R¹ through R⁴ are defined as follows: R¹ is selected from hydrido, hydroxy, carboxyl, alkyl, hydroxyalkyl, haloalkyl, cycloalkyl, cycloalkylalkyl, alkoxyalkyl, alkoxy, alkoxycarbonyl, alkylcarboxyl, aryl, aralkyl, aryloxy, arylthio, alkylthio, amino, amido and carboxyl; R² is selected from alkyl, hydroxyalkyl, haloalkyl, alkoxyalkyl, haloacyl, aryl and aralkyl; R³ is selected from alkyl, hydroxyalkyl, haloalkyl, cycloalkyl, cycloalkylalkyl, alkoxyalkyl, alkoxy, aryl, aralkyl, aryloxy, alkylthio, amino, amido and carboxyl; R⁴ is selected from hydrido, alkyl, hydroxyalkyl, haloalkyl, cyano, cycloalkyl, cycloalkylalkyl, cycloalkylcarbonyl, alkenylalkyl, alkoxyalkyl, aryl, aralkyl, and carboxyl; wherein any of the foregoing R¹, R², R³ and R⁴ substituents having a substitutable position may be substituted with one or more groups selected from hydroxy, halo, alkyl, cycloalkyl, aryl and aralkyl and wherein the dashed line between the 17- and 18-position carbon atoms of the carbon-carbon bridge connecting the 6-and 14-position carbon atoms of the (+)-thevinone intermediate (c) represents a single bond or a double dond. 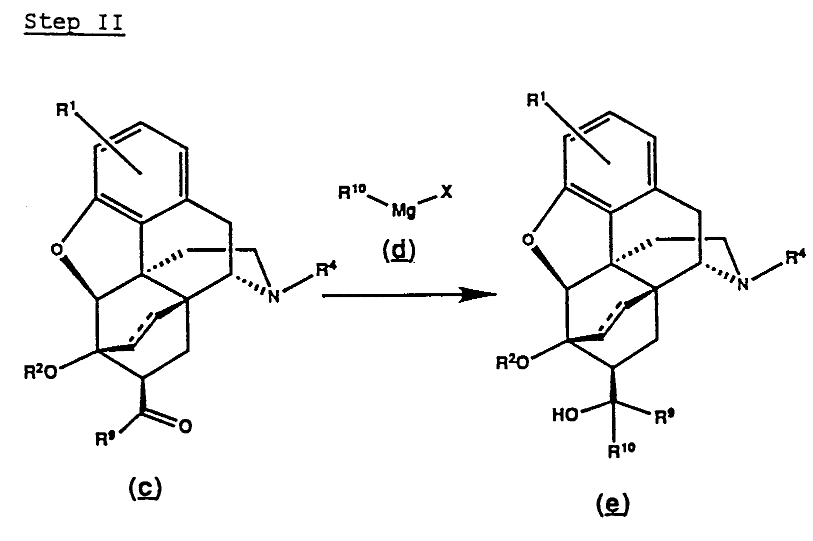
In Step II of the general procedure, the (+)-thevinone-type intermediate (c) is converted to (+)-thevinol-type compound (e). Conversion is effected by use of a Grignard reagent (d) wherein X is a halide, typically bromide or iodide, and wherein R¹⁰ is a precursor residue for inclusion within the R³ substituent of end-product formed in Step II and as shown in Formula I. Typically, R¹⁰ is a linear or branched alkyl group, preferably lower alkyl such as methyl, ethyl, n-propyl, isopropyl, n-butyl, isobutyl, sec-butyl or tert-butyl. Substituents R¹ through R⁴ are as defined in Step I.

Compounds (c) and (e) of Step II fall within the scope of Formula I and would be expected to have non-narcotic, antitussive properties. Products (e) of Step II may also be used as intermediates to form other non-addictive, antitussive compounds within the scope of Formula I. For example, in the procedures of Examples I-XVI which follow, methods are described for preparation of several specific compounds within Formula I. These detailed preparations fall within the scope of, and serve to exemplify, the above-described General Synthetic Procedures all of which form part of the invention. These Examples I-XVI are presented for illustrative purposes only and are not intended as a restriction on the scope of the invention. All parts are by weight unless otherwise indicated.

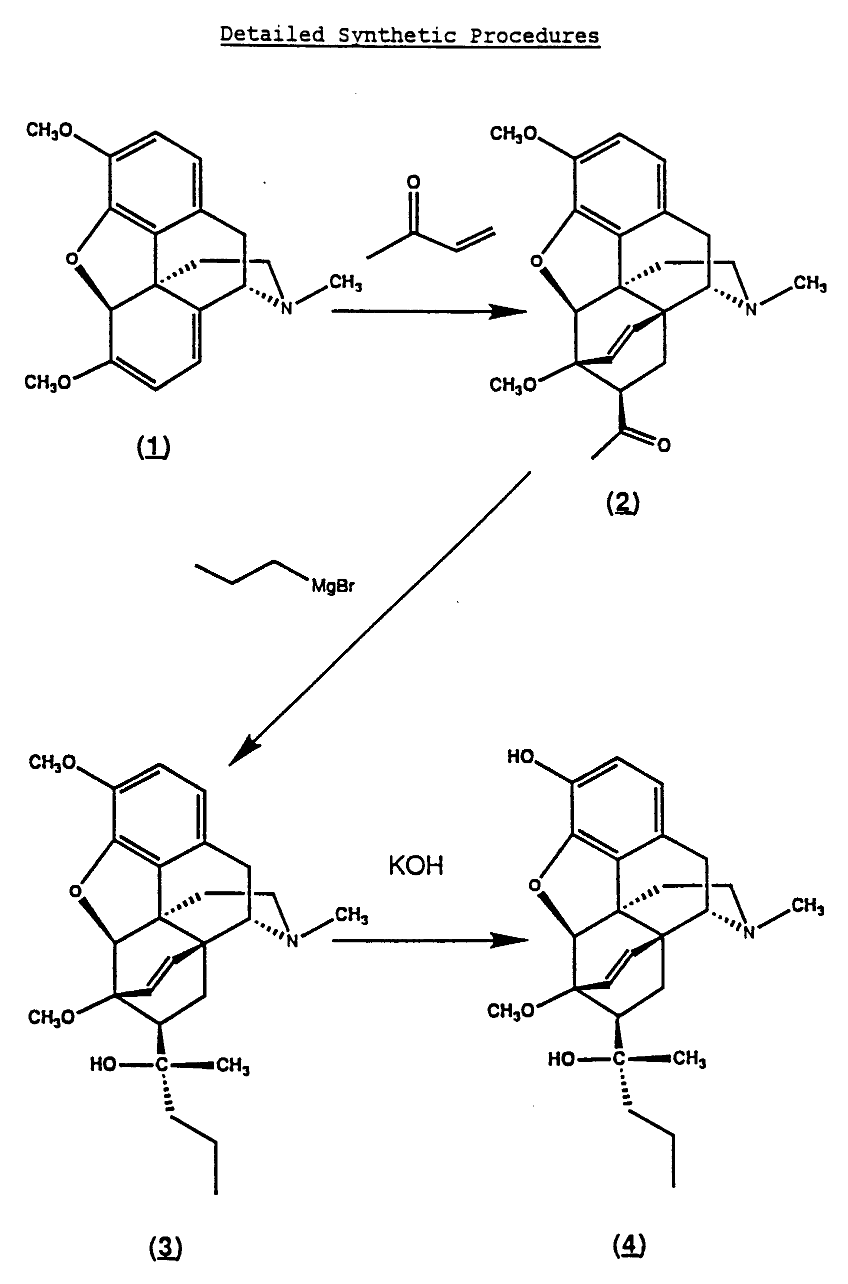

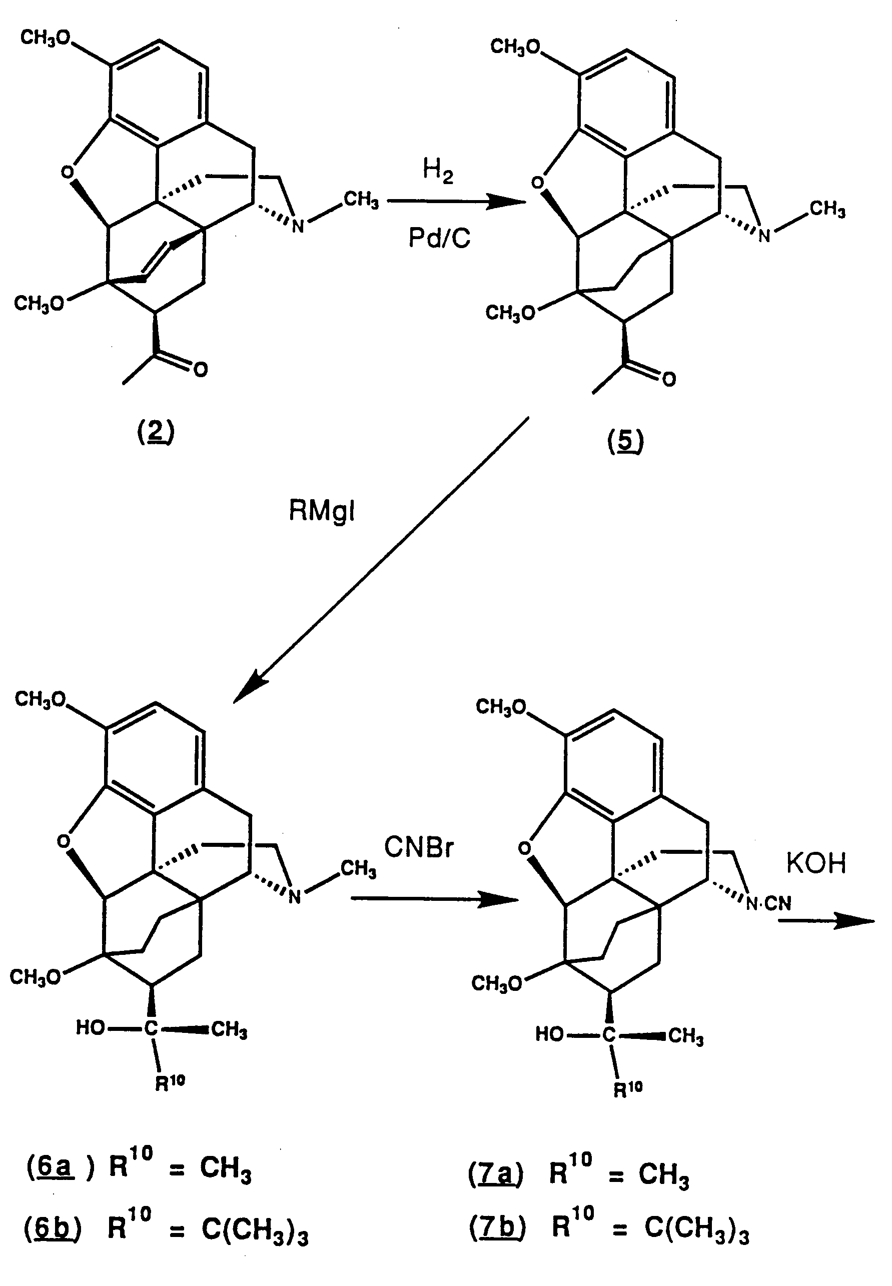

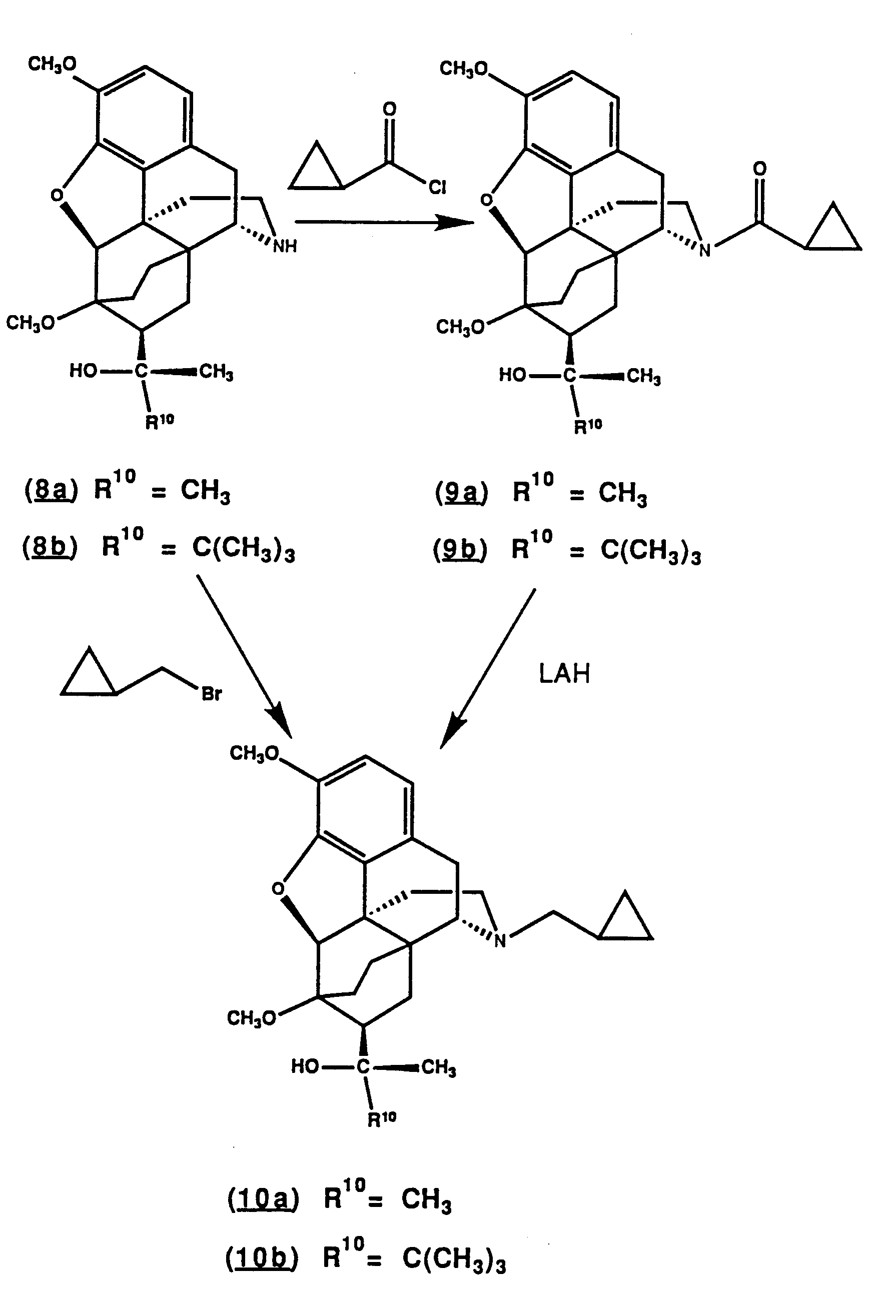

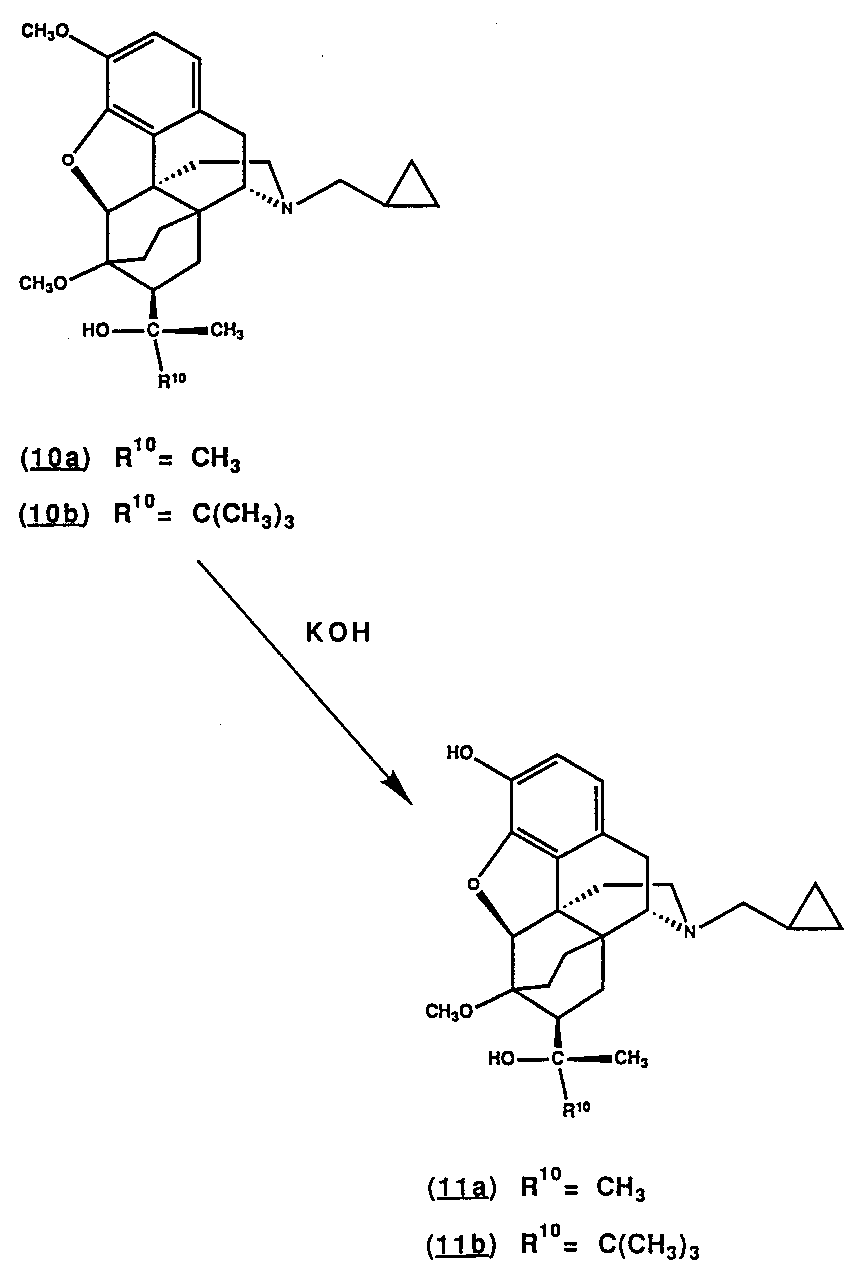

### Example I

### Synthesis of (+)-thevinone (2)

(+)-Thebaine (1) (10 g, 32 mmol, obtained using methods described in U.S. Patent No. 4,521,601) was heated at 100°C and stirred under an inert atmosphere with freshly distilled methyl vinyl ketone (30 mL, 371 mmol) for 1.5 h. The excess methyl vinyl ketone was then removed by distillation under reduced pressure, followed by the addition and removal, in vacuo, of 20 mL of methanol. The residue was dissolved in 15 mL of boiling methanol and cooled to 0°C. The resulting crystalline solid was collected by filtration and recrystallized from methanol to yield 10.6 g (86%) of colorless, crystalline solid, mp (HCl salt) 259-260°C; IR (film) 1715 cm⁻¹; NMR consistent with desired structure; MS m/z 382 (M+1). Anal. Calcd. for C₂₃H₂₇NO₄.HCl: C, 66.26; H, 6.53; N, 3.36. Found: C, 66.14; H, 6.79; N, 3.32.

### Example II

### Synthesis of (+)-19-propylthevinol (3)

Magnesium (1.65 g, 75.9 mmol) was heated under reflux with 11 mL of anhydrous diethyl ether under an inert atmosphere. A solution of 1-bromopropane (6.7 mL, 75.9 mmol) in 36 mL of anhydrous diethyl ether was added dropwise over a 1-h period to the stirred mixture. The resulting suspension was heated under reflux for 45 min. Then a solution of (+)-thevinone (2)(11 g, 28 mmol) in 28 mL of anhydrous benzene was added dropwise over a 20-min. period. The mixture was heated under reflux for an additional 30 min. Then the reaction flask was cooled to 0°C in an ice bath. The cooled solution was cautiously decomposed by the addition of 15 mL of a saturated aqueous ammonium chloride solution. The upper (organic) phase was removed by decantation and the residual white gum was triturated twice with 25 mL of benzene. The combined organic layers were back-washed once with 15 mL of water. The combined organic layers were then dried over magnesium sulfate, filtered, and concentrated in vacuo to obtain a white foam which was crystallized from absolute ethanol to yield 5.6 g (46%) of colorless, crystalline solid, mp 175-176°C; IR and NMR consistent with desired structure; MS m/z 426 (M+1), 408 (M+1 - H₂O). Anal. Calc'd. for C₂₆H₃₅NO₄.C₂H₂O₄: C, 65.23; H, 7.23; N, 2.72. Found: C, 65.12; H, 7.29; N, 2.75.

### Example III

### Synthesis of (+)-etorphine (4)

A solution of potassium hydroxide (21.6 g, 385 mmol) in 57 mL of diethylene glycol was stirred and heated to 200°C under an inert atmosphere. (+)-19-Propylthevinol (3) (3 g, 7 mmol) was added and the resulting mixture was heated for 17 h. The reaction flask was cooled to 25°C and the contents of the flask was poured into 540 mL of water. A saturated solution of ammonium chloride was added until precipitation appeared to be complete. The mixture was cooled at 5°C for 1 h, then the precipitated solid was collected by filtration and washed exhaustively with water until a negative silver nitrate test for chloride ion was obtained. The collected solid was dissolved in 120 mL of chloroform and shaken with 50 mL of water, then the resulting emulsion was filtered through celite filter aid. The organic and aqueous layers were separated and the water layer was extracted with an additional 25 mL of chloroform. The combined chloroform layers were then back-extracted once with 25 mL of water and the chloroform was removed in vacuo to obtain a tan-colored solid. The solid was recrystallized form a mixture of 2-ethoxyethanol and water (2:1) to obtain 1.6 g (56%) of colorless, crystalline solid, mp 215.5-216.5°C (HCl salt, 259-260°C, decomp.); IR and NMR consistent with the desired structure; α 25 (CHCl₃; c=1.003) = (+) 170.9; MS, m/z 412 (M+1), 394 (M+1-H₂O). Anal. Calc'd for C₂₅H₃₂NO₄.HCl: C, 67.03; H, 7.65; N, 3.13. Found: C, 66.77; H, 7.68; N, 3.11.

### Example IV

### Synthesis of the (+)-enantiomer of 7α-acetyl-6,14-endo-ethanotetrahydrothebaine [(+)-dihydrothevinone 5].

A solution of (+)-thevinone (2) (10.3 g, 27.0 mmol) in 300 mL of ethanol was shaken on a Parr apparatus with 10% palladium on charcoal (1.3 g) under hydrogen at 58 psi and 50 °C for 10 h. The mixture was filtered and the solvent was removed from the filtrate under reduced pressure. The residue was crystallized from ethanol to obtain 9.55 g (92%) of colorless, crystalline solid, mp 136-138 °C; IR and NMR data consistent with proposed structure; MS m/z = 384 (M + 1). Anal. calc'd. for C₂₃H₂₉NO₄: C, 72.04; H, 7.62; N, 3.65. Found: C, 71.97; H, 7.63; N, 3.63.

### Example V

### Synthesis of the (+)-enantiomer of 7α-(1-hydroxy-1-methylethyl)-6,14-endo-ethanotetrahydrothebaine (6a).

A solution of (+)-dihydrothevinone (5) (4.0 g, 10 mmol) in 270 mL of anhydrous benzene was added slowly to a stirred, refluxing solution of methylmagnesium iodide, prepared from magnesium (0.68 g, 28 mmol) and methyl iodide (1.7 mL , 28 mmol) in 40 mL of anhydrous ether. The mixture was stirred under reflux for 2 h, then the cooled mixture vigorously stirred with a saturated solution of ammonium chloride. The organic layer was separated and the aqueous layer extracted three times with 25 mL of benzene. The combined organic extracts were washed once with 25 mL of brine, dried over sodium sulfate, and concentrated in vacuo to obtain a white foam which was crystallized from aqueous ethanol to obtain 2.7 g (64%) of colorless, crystalline solid, mp 139-140 °C; IR and NMR data consistent with the proposed structure; MS m/z 400 (M + 1), 382 (M + 1- H₂O). Anal. calc'd. for C₂₄H₃₃NO₄: C, 72.15; H, 8.33; N, 3.51. Found: C, 71.98; H, 8.42; N, 3.44.

### Example VI

### Synthesis of the (+)-enantiomer of N-cyano-7α-(1-hydroxy-1-methylethyl)-6,14-endo-ethanotetrahydronorthebaine (7a).

A solution of the (+)-enantiomer of 7α-(1-hydroxy-1-methylethyl)-6,14-endo-ethanotetrahydrothebaine (6a) (0.86 g, 2.2 mmol) in 10 mL of anhydrous, alcohol-free chloroform was stirred and heated under reflux with cyanogen bromide (0.32g, 3.0 mmol) for 48 h. The chloroform was evaporated to obtain 0.84 g (95%) of the crude base as a white foam. The foam was pulverized then stirred briskly for 0.5 h in 10 mL of 0.1 N HCl. The solid was collected by filtration, washed exhaustively with water, dried in vacuo, and recrystallized from ethanol to obtain a white, crystalline solid, mp 197-198 °C; IR consistent with desired compound; MS m/z 410 (M).

### Example VII

### Synthesis of the (+)-enantiomer of 7α-(1-hydroxy-1-methylethyl)-6,14-endo-ethanotetrahydronorthebaine (8a).

The (+)-enantiomer of N-cyano-7α-(1-hydroxy-1-methylethyl)-6,14-endo-ethanotetrahydrothebaine (7a) (2.52 g, 6.13 mmol) was heated and stirred at 160-165 ° C under inert atmosphere with potassium hydroxide (3.66 g, 68.7 mmol) in 15 mL of ethylene glycol for 2 h. The mixture was cooled, then diluted with 20 mL of water and extracted three times with 15 mL of benzene. The combined organic layers were washed once with 15 mL of brine, dried over sodium sulfate, and concentrated to obtain 2.19 g (93%) of a brown foam; MS m/z 386 (M + 1), 368 (M + 1 - H₂O). This material was used crude in the next reaction.

### Example VIII

### Synthesis of (+)-enantiomer of N-cyclopropylcarbonyl-7α-(1-hydroxy-1-methylethyl)-6,14-endo-ethanotetrahydronorthebaine (9a)

The (+)-enantiomer of the secondary base 7α-(1-hydroxy-1-methylethyl)-6,14-endo-ethanotetrahydronorthebaine (8a) (0.39 g, 1 mmol) is stirred in 15 mL of anhydrous ether with anhydrous potassium carbonate (0.39 g, 2.8 mmol) and cyclopropane carbonyl chloride (0.10 g, 1 mmol) for 6 h. Water and dilute hydrochloric acid are added, then the organic layer is separated, washed with water, and dried. The organic layer is then concentrated under reduced pressure to obtain the desired product.

### Example IX

### Synthesis of (+)-enantiomer of N-cyclopropylmethyl-7α-(1-hydroxy-1-methylethyl)-6,14-endo-ethanotetrahydronorthebaine (10a)

The (+)-enantiomer of the amide N-cyclopropylcarbonyl-7α-(1-hydroxy-1-methylethyl-6,14-endo-ethanotetrahydronorthebaine (9a) (0.43 g, 1 mmol) is stirred and heated under reflux with lithium aluminum hydride (0.12 g, 5.6 mmol) in 10 mL of anhydrous tetrahydrofuran for 5 h. The excess hydride is then destroyed by the addition of an aqueous solution of ammonium chloride to the cooled mixture and the organic layer is separated. The aqueous layer is extracted with ether and the combined organic solutions are dried and concentrated under reduced pressure to obtain the base.

### Example X

### Synthesis of the (+)-enantiomer of N-cyclopropylmethyl-7α-(1-hydroxy-1-methylethyl)-6,14-endo-ethanotetrahydronorthebaine [(10a), alternate procedure].

A mixture of the (+)-isomer of 7α-(1-hydroxy-1-methylethyl)-6,14-endo-ethanotetrahydronorthebaine (8a) (1.86 g, 4.83 mmol), cyclopropylmethyl bromide (0.45 mL , 4.6 mmol), and sodium bicarbonate (1.24 g, 14.7 mmol) was stirred at 25 °C in 50 mL of dimethylformamide for 48 h. The solution was filtered, then the filtrate was evaporated in vacuo. The residue was partitioned between benzene and water. The organic layer was separated, washed once with brine, dried over sodium sulfate, and concentrated in vacuo to obtain the base as a brown foam. The oxalate salt (1.94 g, 76%) was obtained from acetone as a colorless solid, mp (oxalate salt) 211-212 °C; MS m/z 440 (M + 1), 422 (M + 1 - H₂O).

### Example XI

### Synthesis of the (+)-enantiomer of N-cyclopropylmethyl-7α-(1-hydroxy-1-methylethyl)-6,14-endo-ethanotetrahydronororipavine [(+)-diprenorphine 11a].

A mixture of the (+)-enantiomer of N-cyclopropylmethyl-7α-(1-hydroxy-1-methylethyl)-6,14-endo-ethanotetrahydronorthebaine (10a) (0.83 g, 1.9 mmol) and potassium hydroxide (1.1 g, 20 mmol) in 10 mL of diethylene glycol were heated at 220 °C and stirred under inert atmosphere for 2 h. The reaction mixture was cooled, diluted with 15 mL of water, and extracted three times with 15 mL of benzene. The combined organic layers were washed once with 15 mL of water, dried over sodium sulfate, and concentrated to a tan-colored foam. The HCl salt (0.40 g, 38%) was obtained from diethyl ether, mp (HCl salt) >260 °C; pure by gas and thin-layer chromatography (chloroform: methanol: ammonium hydroxide [90:10:1]); MS m/z 426 (M + 1); Anal. calc'd. for C₂₆H₃₅NO₄Cl.

### Example XII

### Synthesis of the (+)-enantiomer of 7α-(1-(R)-hydroxy-1-tert-butyl-ethyl)-6,14-endo-ethanotetrahydrothebaine (6b)

A commercial solution of 2-chloro-2-methylpropane in ether (Aldrich, 2.0 M, 13 mL, 26 mmol) was concentrated in vacuo at 25 °C to a white residue. The residue was dissolved in 10 mL of anhydrous benzene, the solvent was removed in vacuo at ambient temperature, and the procedure was repeated twice. The residual, waxy solid was dissolved in 100 mL of dry benzene, stirred under inert atmosphere, and cooled with an ice bath. A solution of the (+)-enantiomer of 7α-acetyl-6,14-endo-ethanotetrahydrothebaine (5) (2.57 g, 6.70 mmol) in 40 mL of dry benzene was added dropwise to the cooled solution. After addition was complete, the ice bath was removed and the solution was stirred for 0.5 h. The flask was then cooled again with an ice bath, 100 mL of a saturated ammonium chloride solution was added dropwise, and the resulting solution was extracted twice with 50 mL of benzene. The combined benzene extracts were washed once with brine, dried over sodium sulfate, filtered and concentrated to a white foam. The product was purified by column chromatography (Kieselgel 60 silica; tetrahydrofuran: hexanes: ammonium hydroxide [30: 60: 1]). The resulting solid was recrystallized from aqueous ethanol to obtain 1.86 g (62%) of white, crystalline solid, mp 184 °C; IR consistent with proposed compound; MS m/z 442 (M + 1), 424 (M + 1 - H₂O).

### Example XIII

### Synthesis of the (+)-enantiomer of N-cyano-7α-(1-(R)-hydroxy-1-tert-butyl-ethyl)-6,14-endo-ethanotetrahydronorthebaine (7b).

The title compound was synthezised according to the method for compound 7a, substituting the (+)-enantiomer of 7α-(1-(R)-hydroxy-1-tert-butyl-ethyl)-6,14-endo-ethanotetrahydrothebaine (6b) (0.30 g, 0.68 mmol) for compound 6a.

### Example XIV

### Synthesis of the (+)-enantiomer of 7α-(1-(R)-hydroxy-1-tert-butyl-ethyl)-6,14-endo-ethanotetrahydronorthebaine (8b).

The title compound was synthesized according to the method for compound 8a, substituting the (+)-enantiomer of N-cyano-7α-(1-(R)-hydroxy-1-methyl-tert-butylethyl)-6,14-endo-ethanotetrahydronorthebaine (7b) (2.53 g, 5.56 mmol) for 7a. A tan-colored solid was obtained (2.41 g. 100%), pure by gas and thin-layer chromatography (chloroform: methanol: ammonium hydroxide [90: 10: 1]), MS m/z 428 (M + 1). This compound was used crude in the next reaction.

### Example XV

### Synthesis of (+)-enantiomer of N-cyclopropylcarbonyl-7α-(1-(R)-hydroxy-1-tert-butylethyl)-6,14-endo-ethanotetrahydronorthebaine (9b)

The title compound is synthesized according to the method for compound (9a), substituting the (+)-enantiomer of 7α-(1-hydroxy-1-tert-butylethyl)-6,14-endo-ethanotetrahydronorthebaine (8b) (0.43 g, 1 mmol) for compound 8a.

### Example XVI

### Synthesis of (+)-enantiomer of N-cyclopropylmethyl-7α-(1-(R)-hydroxy-1-tert-butylethyl)-6,14-endo-ethanotetrahydronorthebaine

The title compound is synthesized according to the method for compound (10a), substituting the (+)-enantiomer of N-cyclopropylcarbonyl-7α-(1-hydroxy-1-tert-butylethyl)-6,14-endo-ethanotetrahydronorthebaine (9b) (0.47 g, 1 mmol) for compound 9a.

### Example XVII

### Synthesis of the (+)-enantiomer of N-cyclopropylmethyl-7α-(1-(R)-hydroxy-1-tert-butylethyl)-6,14-endo-ethanotetrahydronorthebaine (10b)

The title compound was synthesized according to the method for 10a, substituting the (+)-enantiomer of 7α-(1-(R)-hydroxy-1-tert-butylethyl)-6,14-endo-ethanotetrahydronorthebaine (8b) (0.69 g, 1.62 mmol) for 8a. A tan-colored powder (0.70 g, 90%) was isolated. The oxalate salt was obtained from ether, mp (oxalate salt) 161-163 °C; pure by gas and thin-layer chromatography (chloroform: methanol: ammonium hydroxide [90: 10: 1]); MS m/z 482 (M + 1).

### Example XVIII

### Synthesis of the (+)-enantiomer of N-cyclopropylmethyl-7α-(1-(R)-hydroxy-1-tert-butylethyl)-6,14-endo-ethanotetrahydronororipavine [(+)-buprenorphine 11b].

The title compound was synthesized according to the method for compound 11a, substituting the (+)-isomer of N-cyclopropylmethyl-7α-(1-(R)-hydroxy-1-tert-butylethyl)-6,14-endo-ethanotetrahydronorthebaine (10b) (0.42 g, 0.87 mmol) for 10a. A brown oil was obtained (0.40 g, 98%) which was purified by chromatography (Kieselgel 60 silica; tetrahydrofuran: hexanes: ammonium hydroxide [35: 65: 1]); HCl salt forms from diethyl ether; mp (free base) 210-211 °C; MS m/z 468 (M + 1). Anal calc'd. for C₂₉H₄₁NO₄: C, 74.48; H, 8.84; N, 3.00. Found: C, 74.56; H, 8.87; N, 3.03.

### BIOLOGICAL EVALUATION

Compounds of the invention were evaluated for antitussive properties and opiate properties by in vitro and in vivo assays. The antitussive properties were determined by the ability of compound of the invention to inhibit cough response to an irritant aerosol administered to guinea pigs. The opiate properties were investigated by the determining ability of the compounds of the invention to inhibit specific opiate binding in vitro and by the ability of animals to discriminate between known opiates [such as (-)-morphine] and compounds of the invention.

### Antitussive Assay

In these studies, adult male Dunkin Hartley guinea pigs (350-400 g), obtained from David Hall Company (Berton-on-Trent, Stafordshire), were group housed with food and water ad libitum. Eighteen hours prior to testing, the animals were deprived of food but not water. Saline vehicle or solutions of compound of the invention were administered subcutaneously in a volume of 2 ml/kg (except 30 mg/kg dose of (+)-etorphine at 5 ml/kg, s.c.) or p.o. by gavage tube in a volume of 10 ml/kg. One hour after treatment with the invention compound or saline vehicle, the heads of the animals were positioned in a cylindrical glass chamber (length = 15 cm, internal diameter = 9 cm) with a rubber collar seal at the aperture. The guinea pigs were then exposed for 10 min to a continuous aerosol of 7.5% aqueous-citric acid generated by compressed air at low pressure in a Wright nebulizer. Animals generally responded with spasms of 2-3 coughs per minute. The number of elicited coughs were counted for the first 5 minutes of exposure and for the total 10 minutes of exposure to the citric acid aerosol. The number of coughs in drug-treated animals was subtracted from the number of coughs in saline-treated control animals for each of the 5 and 10 minute periods of exposure. This number was divided by the total number of coughs by saline-treated control animals in the respective periods and multiplied by 100% to determine percent inhibition of cough. Results are reported in Table I.

The data in Table I, and illustrated for the 10 min test period in Figure 1, demonstrate that (+)-etorphine inhibited cough induced by citric acid aerosol in conscious guinea pigs. The inhibition was dose-related between 2.76 and 27.6 mg/kg after s.c. treatment and did not appear to have plateaued at the highest dose tested. The standard narcotic antitussive, (-)-codeine, produced a 46% inhibition of cough at the 10 mg/kg dose s.c. and comparable inhibition after oral dosing of 10 mg/kg. As indicated in the lower half of Table I, (+)-etorphine was more potent after oral administration than after s.c. treatment. By oral route, (+)-etorphine was as effective at 2.76 mg/kg as (-)-codeine was at 10 mg/kg.

**Table I**

| Inhibition of Cough in Guinea Pigs | | | | |
|---|---|---|---|---|
| Group | Treatment | Dose (mg free base/kg) | % Inhibition of Coughing | |
| | | | 5 min | 10 min |
| Subcutaneous Administration | | | | |
| 17 | (+)-etorphine | 2.76 | 0 | 2.9 |
| 18 | " | 9.2 | 34.4*** | 28.2*** |
| 19 | " | 27.6 | 68.8*** | 63.1*** |
| 20 | (-)-codeine | 10.0 | 46.2*** | 46.1*** |

| Oral Administration | | | | |
|---|---|---|---|---|
| 5 | (+)-etorphine | 0.92 | 0 | 9.8 |
| 6 | " | 2.76 | 34.4** | 37.6*** |
| 7 | " | 9.2 | 55.2*** | 54.1*** |
| 8 | (-)-codeine | 10.0 | 42.7*** | 38.7*** |

| | | | | |
|---|---|---|---|---|
| * p<0.05 versus control coughing | | | | |
| ** p<0.01 versus control coughing | | | | |
| *** p<0.001 versus control coughing | | | | |

In summary by s.c. administration, (+)-etorphine appeared comparable in potency to (-)-codeine. By p.o. administration, (+)-etorphine was 3 times more potent than (-)-codeine.

### Opiate Binding Assay

Male Sprague-Dawley rats [COBS CD(SD)BR, Charles River Laboratories, Portage, MI] were sacrificed by cervical dislocation and their brains, minus cerebellum, were removed and placed in a beaker containing 30.0 ml of 5 mM TRIS-HCl buffer (pH 7.4 @ 4°c). The brains were blotted dry on a paper towel, weighed and their total weight recorded. All subsequent steps were performed at 0 to 4°C. Brains were transferred into an Erlenmeyer flask into which 4 volumes (4 x total weight) of 5 mM TRIS-HCl buffer (pH 7.4 @ 0°C) was added. Brains were cut into 0.5 cm pieces, transferred into a 55 ml grinding vessel, and homogenized (10 strokes at 800 to 1000 rpm). Homogenate was poured into a flask and 26 volumes (26 ml x total weight) of 5 mM TRIS-HCl buffer (pH 7.4 @ 0°C) was added to yield a final 30 volumes of buffer. The homogenate was then divided into 50 ml polyethylene (PE) centrifuge tubes and spun at 1000 x g for 10 min. The S₁ supernatants were decanted into fresh 50 ml PE centrifuge tubes and spun at 30,000 x g for 15 min. The P₁ pellets were resuspended in 4 volumes 5 mM TRIS-HCl buffer (pH 7.4 @ 0°C), adjusted to 30 volumes with buffer, total volume recorded, and a 3.0 ml aliquot transferred into a 3.5 ml Nunc Cryotube to be frozen at -20°C. [All subsequent resuspensions were performed in this manner.] The P₂ pellets were resuspended and allowed to swell for 30 min at 0°C, after which a 30,000 x g (15 min) centrifugation was performed. The once washed P₂ pellets were then resuspended and allowed to swell an additional 30 min at 0°C and centrifuged for 15 min at 30,000 x g. The twice washed P₂ pellets were resuspended using 50 mM TRIS-HCl buffer (pH 7.4 @ 35°C) without diluting to 30 volumes. The total volume was recorded and the resuspended pellets were transferred into 3.5 ml Nunc tubes which were frozen at -70°C. Protein concentrations were determined on all fractions using the micro-biuret protein assay. A 50 mM TRIS-HCl buffer (pH 7.4 @ 37°C) was used unless stated otherwise. Membrane preparations were thawed at room temperature and diluted with buffer to a final concentration of 0.625 mg protein/ml. After all additions, the final amount of protein in each assay tube was 0.5 mg. (+)-Etorphine was solubilized in 100% ethyl-alcohol to yield a 1.0 mM solution. This stock solution was then diluted with buffer to yield 2 concentrations of 0.1 mM & 1.0 »M (10.0 »M & 100.0 nM final in assay tubes). Binding assay was conducted in triplicate with each assay tube containing 0.8 ml membrane homogenate, 0.1 ml ³H-ligand [1.0 nM ³H-D-Ser²-Leu-enkephalin-Thr (DSLET) for δ, 2 nM ³H-[D-Ala², NMe-Phe⁴,Gly-ol-enkephalin] (DAMPGO) for »], and 0.1 ml of (+)-etorphine at the desired concentration. Each sample was incubated for 60 min. at 37°C. The assay was terminated by the addition 10.0 ml of ice-cold 50 mM TRIS-HCl buffer (pH 7.4 @ 4°C) and rapid filtration over Whatman GF/B glass fiber filters utilizing a Brandel Cell Harvester. Filters were dried at 80°C for 30 min. and transferred to 20 ml scintillation vials to which 10 ml PCS (Amersham) was added. Radioactivity was determined using a Tracor Mark III Scintillation counter at an efficiency of about 40%. Specific binding was defined as the difference between the amount of radioactivity bound in the absence and the presence of 10 »M levorphanol. Assays were performed at 35°C for 60 min. The ligands used for specific opiate binding were DSLET at 1 nM for delta and DAMPGO at 2 nM for mu binding. Results are reported in Table II.

**Table II**

| Mu and Delta Opiate Binding Affinities of (-)- Morphine, D-Thr²-Leu-Enkephalin-Thr (DTLET), and (+)-Etorphine. | | | | |
|---|---|---|---|---|
| Compound | IC₅₀ Delta (nM) | IC₅₀ Mu (nM) | Kᵢ Delta | Kᵢ Mu |
| Morphine | 362.0 | 64.9 | 36.9 | |
| DTLET | 2.7 | 184.0 | 1.2 | 104.8 |
| (+)-Etorphine | - * | - * | | |

| | | | | |
|---|---|---|---|---|
| * 31% displacement at 10 million nM | | | | |

As indicated by the binding data in Table II, (+)-etorphine was unable to displace the binding of radioactively labeled ligands specific for the opiate binding sites associated with narcotic properties of opiates. These in vitro data indicate that (+)-etorphine is inactive as a classical opiate narcotic. This finding is further supported by in vivo results where (+)-etorphine, unlike its (-)-isomer, was found to be totaly inactive in drug discrimination tests in animals trained to (-)- morphine. Whereas (-)-etorphine dose-dependently generalized to the morphine stimulus and produced 100% morphine lever responding, (+)-etorphine did not generalize to the morphine stimulus nor did it have any marked effects on response rates up to 30 mg/kg.

### Discriminative Stimulus Assay

Twelve male Long Evans hooded rats, which were previously trained to discriminate 3.0 mg/kg s.c. (-)-morphine from water vehicle, in a two-lever, food-reinforced operant test, were provided 15-min daily (Mon-Fri) experimental sessions. Test days were scheduled for Tuesdays and Fridays. A rat was tested on a scheduled test day provided it had ≧ 80% correct responses and had chosen the correct lever for completing the initial fixed ratio during the immediately preceding vehicle and drug training session. If a rat was unable to be tested on a scheduled test day because it had not met criteria it was given a training session instead. Other general conditions of training and testing were conducted as published previously [P. M. Beardsley et al, J. Pharmacol. Exp. Ther., 241, 159-165 (1987)]. Four rats were used to evaluate each test drug. Each group of four rats was tested with the (+)- and (-)-isomer of the same drug.

The following doses of the etorphine isomers were tested: 0.0003, 0.001, 0.003, and 0.01 mg/kg of (-)- etorphine; 0.1, 1.0, 10.0 and 30.0 mg/kg (+)-etorphine. In addition, water vehicle and the 3.0 mg/kg training dose of (-)-morphine were tested. All injections were given subcutaneously 30 min prior to the start of the experimental session.

The two main dependent variables in these procedures is the percent of morphine lever responding and overall response rates. Percent morphine-lever responding was calculated by dividing the total number of lever responses emitted at the morphine lever by the total number of lever responses emitted at both levers with this quotient then multiplied by 100. Normally, when the training dose of (-)- morphine is administered, rats trained to discriminate the morphine from water will respond nearly exclusively at the morphine-designated lever. That is the percent morphine-lever responding will be near 100%. Conversely, when water vehicle is administered, morphine-lever responding will be near 0%. If a test drug generates greater than 50% morphine-lever responding, the inference would be that it possesses subjective effects similar to those of morphine. Response rates are calculated for each rat by dividing the total number of responses emitted at both levers by 900 sec, the duration of the test session. Mean percent morphine-lever responding and mean response rates were calculated by averaging individual rat values. Results are reported in Table III.

**Table III**

| Mean Percent Morphine-Lever Responding (S.E.M.) | | | | | |
|---|---|---|---|---|---|
| Water | (-)-Morphine (3.0 mg/kg) | (-)-Etorphine (mg/kg, s.c.) | | | |
| | | 0.0003 | 0.001 | 0.003 | 0.01 |
| 0.13 | 99.96 | 0.00 | 74.97 | 100.00 | NR |
| (0.10) | (0.04) | (0.00) | (24.99) | (0.00) | NR |

| Water | (-)-Morphine (3.0 mg/kg) | (+)-Etorphine (mg/kg, s.c.) | | | |
|---|---|---|---|---|---|
| | | 0.1 | 1.0 | 10.0 | 30.0 |
| 0.01 | 100.00 | 0.11 | 0.00 | 0.02 | 0.06 |
| (0.01) | (0.00) | (0.09) | (0.00) | (0.02) | (0.04) |

The data in Table III show that (-)-etorphine generalized to the morphine stimulus (i.e., produced morphine-lever responding) in a dose-dependent manner. At 0.003 mg/kg, 100% morphine-lever responding was produced. (-)-Etorphine also suppressed responding in a dose-dependent manner. At 0.01 mg/kg, responding was totally suppressed. Data in Table III also show that (+)-etorphine did not generate morphine-lever responding up to doses 10,000 times greater than those producing 100% morphine-lever responding by the (-)-isomer. (+)-Etorphine also did not have marked effects on response rates up to 30 mg/kg, the highest dose tested.

The data in Tables I-III indicate that (+)-etorphine possesses a pharmacological profile making it suitable as a therapeutic antitussive agent without the narcotic liability, such as respiratory depression, constipation and addiction, typically associated with opiate compounds.

### Compositions of the Invention

Also embraced within this invention is a class of pharmaceutical compositions comprising one or more compounds of Formula I described above in association with one or more non-toxic, pharmaceutically acceptable carriers and/or diluents and/or adjuvants (collectively referred to herein as "carrier" materials) and, if desired, other active ingredients. The compounds of the present invention may be administered by any suitable route, preferably in the form of a pharmaceutical composition adapted to such a route, and in a dose effective for the treatment intended. Therapeutically effective doses of the compounds of the present invention required to prevent or suppress the cough reflex action are readily ascertained by one of ordinary skill in the art. The compounds and compositions may, for example, be administered intravascularly, intraperitoneally, subcutaneously, intramuscularly or topically.

For oral administration, the pharmaceutical compositions may be in the form of, for example, a tablet, capsule, suspension or liquid. The pharmaceutical compositions is preferably made in the form of a dosage unit containing a particular amount of the active ingredient. Examples of such dosage units are tablets or capsules. These may with advantage contain an amount of active ingredient from about 1 to 250 mg, preferably from about 25 to 150 mg. A suitable daily dose for a human may vary widely depending on the condition of the patient and other factors. However, a dose of from about 0.1 to 3000 mg/kg body weight, particularly from about 1 to 100 mg/kg body weight, may be appropriate.

The active ingredient may also be administered by injection as a composition wherein, for example, saline, dextrose solutions or water may be used as a suitable carrier. A suitable daily dose is from about 0.1 to 100 mg/kg body weight injected per day in multiple doses depending on the disease being treated. A preferred daily dose would be from about 1 to 30 mg/kg body weight. Compounds indicated for effective therapy will preferably be administered in a daily dose generally in a range from about 0.1 mg to about 100 mg per kilogram of body weight per day. A more preferred dosage will be a range from about 1 mg to about 100 mg per kilogram of body weight. Most preferred is a dosage in a range from about 1 to about 50 mg per kilogram of body weight per day. A suitable dose can be administered in multiple, sub-doses per day. These sub-doses may be administered in unit dosage forms. Typically, a dose or sub-dose may contain from about 1 mg to about 100 mg of active compound per unit dosage form. A more preferred dosage will contain from about 2 mg to about 50 mg of active compound per unit dosage form. Most preferred is a dosage form containing from about 3 mg to about 25 mg of active compound per unit dose.

The dosage regimen for suppressing a cough condition with the compounds and/or compositions of this invention is selected in accordance with a variety of factors, including the type, age, weight, sex and medical condition of the patient, the severity of the cough, the route of administration, and the particular compound employed, and thus may vary widely.

For therapeutic purposes, the compounds of the invention are ordinarily combined with one or more adjuvants appropriate to the indicated route of administration. If administered per os, the compounds may be admixed with lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinylpyrrolidone, and/or polyvinyl alcohol, and than tableted or encapsulated for convenient administration. Such capsules or tablets may contain a controlled-release formulation as may be provided in a dispersion of active compound in hydroxy-propylmethyl cellulose. Formulations for parenteral administration may be in the form of aqueous or non-aqueous isotonic sterile injection solutions or suspensions. These solutions and suspensions may be prepared from sterile powders or granules having one or more of the carriers or diluents mentioned for use in the formulations for oral administration. The compounds may be dissolved in water, polyethylene glycol, propylene glycol, ethanol, corn oil, cottonseed oil, peanut oil, sesame oil, benzyl alcohol, sodium chloride solutions, and/or various buffer solutions. Other adjuvants and modes of administration are well and widely known in the pharmaceutical art. Appropriate dosages, in any given instance, of course depend upon the nature and severity of the condition treated, the route of administration, including the weight of the patient.

Representative carriers, diluents and adjuvants include for example, water, lactose, gelatin, starches, magnesium stearate, talc, vegetable oils, gums, polyalkylene glycols, petroleum jelly, etc. The pharmaceutical compositions may be made up in a solid form such as granules, powders or suppositories or in a liquid form such as solutions, suspensions or emulsions. The pharmaceutical compositions may be subjected to conventional pharmaceutical operations such as sterilization and/or may contain conventional pharmaceutical adjuvants such as preservatives, stabilizers, wetting agents, emulsifiers, buffers, etc.

Although this invention has been described with respect to specific embodiments, the details of these embodiments are not to be construed as limitations. Various equivalents, changes and modifications may be made without departing from the spirit and scope of this invention, and it is understood that such equivalent embodiments are part of this invention.

## Claims (Claims for the following Contracting State(s): AT, BE, CH, DE, DK, FR, GB, IT, LI, LU, NL, SE)

1. A compound of the formula 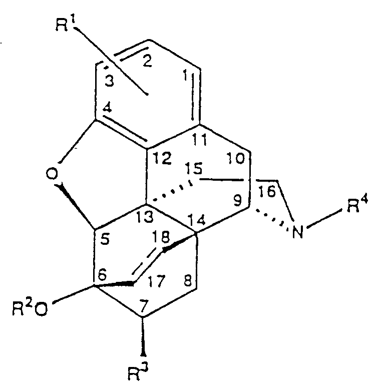 wherein R¹ is selected from hydrido, hydroxy, alkyl, hydroxyalkyl, haloalkyl, cycloalkyl, cycloalkylalkyl, alkoxyalkyl, alkoxy, alkoxycarbonyl, alkylcarboxyl, aryl, aralkyl, aryloxy, arylthio, alkylthio, amino, amido and carboxyl; wherein R² is selected from hydrido, alkyl, hydroxyalkyl, haloalkyl, alkoxyalkyl, haloacyl, aryl and aralkyl; wherein R³ is selected from alkyl, hydroxyalkyl, haloalkyl, cycloalkyl, cycloalkylalkyl, alkoxyalkyl, alkenylalkyl, alkoxy, aryl, aralkyl, aryloxy, alkylthio, amino, amido, carboxyl and 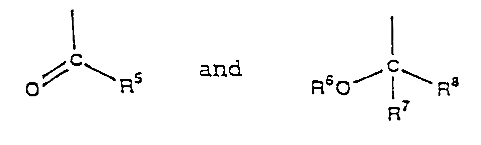 wherein each of R⁵, R⁶, R⁷ and R⁸ is independently selected from hydrido, alkyl,cycloalkyl, phenyl and phenalkyl; wherein R⁴ is selected from hydrido, alkyl, hydroxyalkyl, haloalkyl, cyano, cycloalkyl, cycloalkylalkyl, cycloalkylcarbonyl, alkenylalkyl, alkoxyalkyl, aryl, aralkyl, and carboxyl; wherein any of the foregoing R¹, R², R³ and R⁴ substituents having a substitutable position may be substituted with one or more groups selected from hydroxy, halo, alkyl, cycloalkyl, aryl and aralkyl; and wherein the dashed line between the 17- and 18-position carbon atoms of the carbon-carbon bridge connecting the 6- and 14-position carbon atoms represents a single bond or a double bond; or a pharmaceutically-acceptable salt thereof.

2. Compound of Claim 1 wherein R¹ is selected from hydrido, hydroxy, alkyl, hydroxyalkyl, alkoxyalkyl, alkoxy, alkoxycarbonyl, alkylcarboxyl, phenoxy and phenylthio; wherein R² is selected from hydrido, alkyl, hydroxyalkyl, alkoxyalkyl, phenyl and phenalkyl; wherein R³ is selected from alkyl, hydroxyalkyl, haloalkyl, cycloalkyl, cycloalkylalkyl, cycloalkylcarbonyl, alkenylalkyl, alkoxyalkyl and 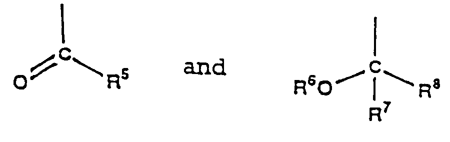 wherein each of R⁵, R⁶, R⁷ and R⁸ is independently selected from hydrido, alkyl, cycloalkyl, phenyl and phenalkyl;
wherein R⁴ is selected from hydrido, alkyl, hydroxyalkyl, haloalkyl, cyano, cycloalkyl, cycloalkylalkyl, cycloalkylcarbonyl, alkenylalkyl, alkoxyalkyl, phenyl and phenalkyl; and wherein any of the foregoing R¹, R², R³ and R⁴ substituents having a substitutable position may be substituted with one or more groups selected from hydroxy, halo, alkyl, cycloalkyl, phenyl and phenalkyl.

3. Compound of Claim 2 wherein R¹ is selected from hydroxy, alkyl, hydroxyalkyl, alkoxyalkyl, alkoxy, alkylcarboxyl and phenoxy; wherein R² is selected from hydrido, alkyl, hydroxyalkyl, alkoxyalkyl, phenyl and phenalkyl; wherein R³ is selected from alkyl, hydroxyalkyl, haloalkyl, cycloalkyl, cycloalkylalkyl, alkoxyalkyl and 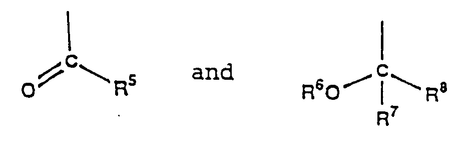 wherein each of R⁵, R⁶, R⁷ and R⁸ is independently selected from hydrido, alkyl, cycloalkyl, phenyl and phenalkyl;
wherein R⁴ is selected from hydrido, alkyl, hydroxyalkyl, cyano, cycloalkyl, cycloalkylalkyl, cycloalkylcarbonyl, allyl, alkoxyalkyl, phenyl and phenalkyl; and wherein any of the foregoing R¹, R², R³ and R⁴ substituents having a substitutable position may be substituted with one or more groups selected from hydroxy, halo, alkyl, cycloalkyl, phenyl and phenalkyl.

4. Compound of Claim 3 wherein R¹ is selected from hydroxy, alkylcarboxyl, alkoxy and phenoxy; wherein R² is selected from hydrido, alkyl, and phenyl; wherein R³ is selected from 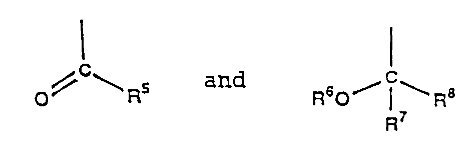 wherein each of R⁵, R⁶, R⁷ and R⁸ is independently selected from hydrido, alkyl, cycloalkyl, phenyl and phenalkyl; wherein R⁴ is selected from hydrido, alkyl, cyano, cycloalkyl, cycloalkylalkyl, cycloalkylcarbonyl, allyl, phenyl, benzyl, phenethyl and phenpropyl; and wherein any of the foregoing R¹, R², R³, R⁴, R⁵, R⁶, R⁷ and R⁸ substituents having a substitutable position may be substituted with one or more groups selected from hydroxy, halo, alkyl and cycloalkyl.

5. Compound of Claim 4 wherein R¹ is selected from hydroxy, alkylcarboxyl, alkoxy and phenoxy; wherein R² is selected from hydrido, alkyl, and phenyl; wherein each of R⁵, R⁶, R⁷ and R⁸ is independently selected from hydrido, alkyl and cycloalkyl; and wherein R⁴ is selected from hydrido, cyano, alkyl, cycloalkyl, cycloalkylalkyl, cycloalkylcarbonyl and allyl.

6. Compound of Claim 5 wherein R¹ is selected from hydroxy, methylcarboxyl, methoxy, ethoxy, n-propoxy, isopropoxy, n-butoxy, isobutoxy, sec-butoxy, tert-butoxy and phenoxy; wherein R² is selected from hydrido, methyl, ethyl, n-propyl, isopropyl, n-butyl, isobutyl, sec-butyl, tert-butyl, acetyl, and phenyl; wherein each of R⁵, R⁶, R⁷ and R⁸ is independently selected from hydrido, methyl, ethyl, n-propyl, isopropyl, n-butyl, isobutyl, sec-butyl, tert-butyl; and wherein R⁴ is selected from hydrido, cyano, methyl, ethyl, n-propyl, isopropyl, n-butyl, isobutyl, sec-butyl, tert-butyl, cyclopropyl, methyl, allyl and cyclopropylcarbonyl.

7. Compound of Claim 6 wherein each of R⁵, R⁷ and R⁸ is selected from methyl, ethyl, n-propyl, isopropyl, n-butyl, isobutyl, sec-butyl and tert-butyl; and wherein R⁶ is hydrido.

8. Compound of Claim 7 wherein R³ is selected from

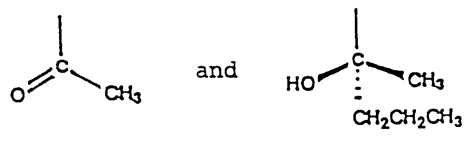

9. Compound of Claim 8 wherein said compound has a double bond between the 17- and 18-position carbon atoms of the carbon-carbon bridge connecting the 6- and 14-position carbon atoms.

10. Compound of Claim 9 which is (+)-enantiomer of 7α-(1-(R)-hydroxy-1-methylbutyl)-6,14-endo-ethenotetrahydrooripavine.

11. Compound of claim 9 which is (+)-enantiomer of 7α-(1-(R)-hydroxy-1-methylbutyl)-6,14-endo-ethenotetrahydrothebaine.

12. Compound of Claim 9 which is (+)-enantiomer of 7α-acetyl-6,14-endo-ethenotetrahydrothebaine.

13. Compound of Claim 8 wherein said compound has a single bond between the 17- and 18-position carbon atoms of the carbon-carbon bridge connecting the 6- and 14-position carbon atoms.

14. Compound of Claim 13 selected from the group consisting of (+)-enantiomer of 7α-acetyl-6,14-endo-ethanotetrahydrothebaine; (+)-enantiomer of 7α-(1-hydroxy-1-methylethyl)-6,14-endo-ethanotetrahydrothebaine; (+)-enantiomer of N-cyano-7α-(1-hydroxy-1-methylethyl)-6,14-endo-ethanotetrahydronorthebaine; (+)-enantiomer of 7α-(1-hydroxy-1-methylethyl)-6,14-endo-ethanotetrahydronororipavine; (+)-enantiomer of N-cyclopropylcarbonyl-7α-(1-hydroxy-1-methylethyl)-6,14-endo-ethanotetrahydronororipavine; and (+)-enantiomer of N-cyclopropylmethyl-7α-(1-hydroxy-1-methylethyl)-6,14-endo-ethanotetrahydronororipavine.

15. Compound of Claim 14 which is (+)-enantiomer of N-cyclopropylmethyl-7α-(1-(R)-hydroxy-1-methylethyl)-6,14-endo-ethanotetrahydronororipavine.

16. Compound of Claim 13 selected from the group consisting of (+)-enantiomer of 7α-(1-hydroxy-1-tert-butyl-ethyl)-6,14-endo-ethanotetrahydrothebaine; (+)-enantiomer of N-cyano-7α-(1-hydroxy-tert-butyl-ethyl)-6,14-endo-ethanotetrahydronorthebaine; and (+)-enantiomer of N-cyclopropylmethyl-7α-(1-hydroxy-1-tert-butyl-ethyl)-6,14-endo-ethanotetrahydronororipavine.

17. Compound of Claim 16 which is (+)-enantiomer of N-cyclopropylmethyl-7α-(1-(R)-hydroxy-1-tert-butyl-ethyl)-6,14-endo-ethanotetrahydronororipavine.

18. A pharmaceutical composition comprising a substantially non-opiate, epoxymorphinan derivative and one or more pharmaceutically-acceptable excipients, said non-opiate epoxymorphinan derivative provided by one or more compounds of the formula according to Claim 1.

19. A pharmaceutical composition according to Claim 18 wherein the non-opiate epoxymorphinan derivative is selected from a compound according to any of claims 2-17.

20. Use of a compound according to Claim 1 for preparing a medicament for suppressing cough in a subject susceptible to or afflicted by cough.

21. Use according to Claim 20 wherein the compound is selected from a compound according to any of claims 2-17.

## Claims (Claims for the following Contracting State(s): ES)

1. A process for preparing a compound of the formula 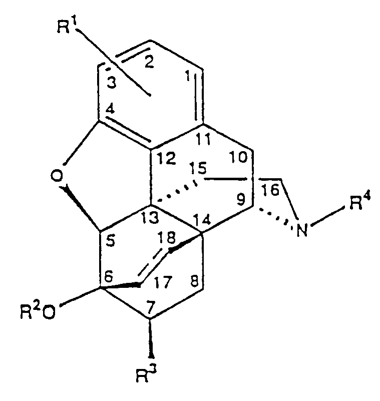 wherein R¹ is selected from hydrido, hydroxy, alkyl, hydroxyalkyl, haloalkyl, cycloalkyl, cycloalkylalkyl, alkoxyalkyl, alkoxy, alkoxycarbonyl, alkylcarboxyl, aryl, aralkyl, aryloxy, arylthio, alkylthio, amino, amido and carboxyl; wherein R² is selected from hydrido, alkyl, hydroxyalkyl, haloalkyl, alkoxyalkyl, haloacyl, aryl and aralkyl; wherein R³ is selected from alkyl, hydroxyalkyl, haloalkyl, cycloalkyl, cycloalkylalkyl, alkoxyalkyl, alkenylalkyl, alkoxy, aryl, aralkyl, aryloxy, alkylthio, amino, amido, carboxyl and 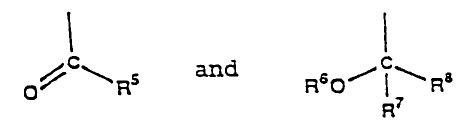 wherein each of R⁵, R⁶, R⁷ and R⁸ is independently selected from hydrido, alkyl, cycloalkyl, phenyl and phenalkyl; wherein R⁴ is selected from hydrido, alkyl, hydroxyalkyl, haloalkyl, cyano, cycloalkyl, cycloalkylalkyl, cycloalkylcarbonyl, alkenylalkyl, alkoxyalkyl, aryl, aralkyl, and carboxyl; wherein any of the foregoing R¹, R², R³ and R⁴ substituents having a substitutable position may be substituted with one or more groups selected from hydroxy, halo, alkyl, cycloalkyl, aryl and aralkyl; and wherein the dashed line between the 17- and 18-position carbon atoms of the carbon-carbon bridge connecting the 6- and 14-position carbon atoms represents a single bond or a double bond; or a pharmaceutically-acceptable salt thereof, characterized in that a compound of the formula (a) 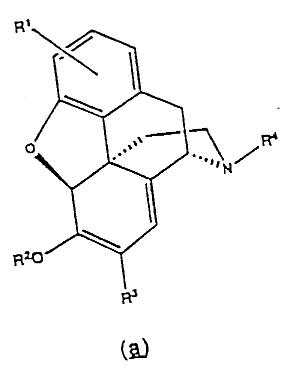 wherein R¹ through R⁴ are as defined above
is reacted under appropriate conditions with a vinyl ketone of the formula (b) 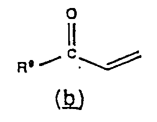 wherein R⁹ is a precursor residue to be included within the R³ substituent of the final product,
thus leading to the compound (c) of the formula 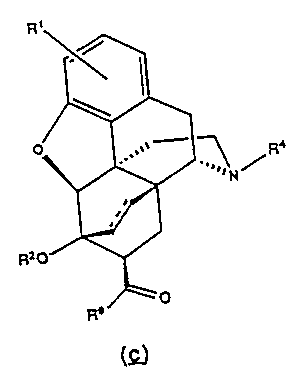 with all the substituents as defined above
said compound (c) then being reacted by use of a Grignard reagent (d) 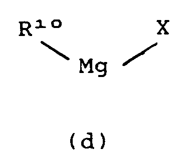 wherein X is a halide and R¹⁰ a precursor residue for inclusion within the R³ substituent of the final product, thus leading to the desired final compounds which optionally may be transformed in a known manner into their respective pharmaceutically-acceptable salts.

2. Process according to Claim 1 wherein R¹ of the compound prepared is selected from hydrido, hydroxy, alkyl, hydroxyalkyl, alkoxyalkyl, alkoxy, alkoxycarbonyl, alkylcarboxyl, phenoxy and phenylthio; wherein R² is selected from hydrido, alkyl, hydroxyalkyl, alkoxyalkyl, phenyl and phenalkyl; wherein R³ is selected from alkyl, hydroxyalkyl, haloalkyl, cycloalkyl, cycloalkylalkyl, cycloalkylcarbonyl, alkenylalkyl, alkoxyalkyl and 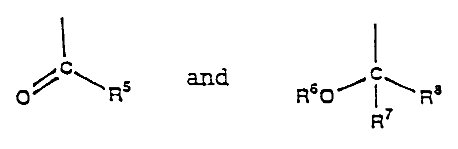 wherein each of R⁵, R⁶, R⁷ and R⁸ is independently selected from hydrido, alkyl, cycloalkyl, phenyl and phenalkyl;
wherein R⁴ is selected from hydrido, alkyl, hydroxyalkyl, haloalkyl, cyano, cycloalkyl, cycloalkylalkyl, cycloalkylcarbonyl, alkenylalkyl, alkoxyalkyl, phenyl and phenalkyl; and wherein any of the foregoing R¹, R², R³ and R⁴ substituents having a substitutable position may be substituted with one or more groups selected from hydroxy, halo, alkyl, cycloalkyl, phenyl and phenalkyl.

3. Process according to Claim 2 wherein R¹ of the compound prepared is selected from hydroxy, alkyl, hydroxyalkyl, alkoxyalkyl, alkoxy, alkylcarboxyl and phenoxy; wherein R² is selected from hydrido, alkyl, hydroxyalkyl, alkoxyalkyl, phenyl and phenalkyl; wherein R³ is selected from alkyl, hydroxyalkyl, haloalkyl, cycloalkyl, cycloalkylalkyl, alkoxyalkyl and 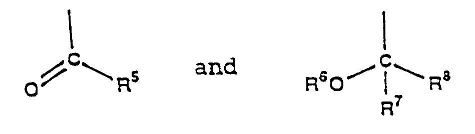 wherein each of R⁵, R⁶, R⁷ and R⁸ is independently selected from hydrido, alkyl, cycloalkyl, phenyl and phenalkyl;
wherein R⁴ is selected from hydrido, alkyl, hydroxyalkyl, cyano, cycloalkyl, cycloalkylalkyl, cycloalkylcarbonyl, allyl, alkoxyalkyl, phenyl and phenalkyl; and wherein any of the foregoing R¹, R², R³ and R⁴ substituents having a substitutable position may be substituted with one or more groups selected from hydroxy, halo, alkyl, cycloalkyl, phenyl and phenalkyl.

4. Process according to Claim 3 wherein R¹ of the compound prepared is selected from hydroxy, alkylcarboxyl, alkoxy and phenoxy; wherein R² is selected from hydrido, alkyl, and phenyl; wherein R³ is selected from 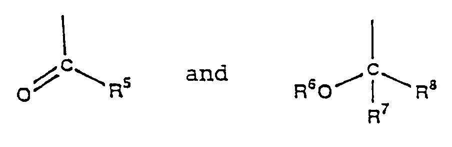 wherein each of R⁵, R⁶, R⁷ and R⁸ is independently selected from hydrido, alkyl, cycloalkyl, phenyl and phenalkyl; wherein R⁴ is selected from hydrido, alkyl, cyano, cycloalkyl, cycloalkylalkyl, cycloalkylcarbonyl, allyl, phenyl, benzyl, phenethyl and phenpropyl; and wherein any of the foregoing R¹, R², R³, R⁴, R⁵, R⁶, R⁷ and R⁸ substituents having a substitutable position may be substituted with one or more groups selected from hydroxy, halo, alkyl and cycloalkyl.

5. Process according to Claim 4 wherein R¹ of the compound prepared is selected from hydroxy, alkylcarboxyl, alkoxy and phenoxy; wherein R² is selected from hydrido, alkyl, and phenyl; wherein each of R⁵, R⁶, R⁷ and R⁸ is independently selected from hydrido, alkyl and cycloalkyl; and wherein R⁴ is selected from hydrido, cyano, alkyl, cycloalkyl, cycloalkylalkyl, cycloalkylcarbonyl and allyl.

6. Process according to Claim 4 wherein R¹ of the compound prepared is selected from hydroxy, methylcarboxyl, methoxy, ethoxy, n-propoxy, isopropoxy, n-butoxy, isobutoxy, sec-butoxy, tert-butoxy and phenoxy; wherein R² is selected from hydrido, methyl, ethyl, n-propyl, isopropyl, n-butyl, isobutyl, sec-butyl, tert-butyl, acetyl, and phenyl; wherein each of R⁵, R⁶, R⁷ and R⁸ is independently selected from hydrido, methyl, ethyl, n-propyl, isopropyl, n-butyl, isobutyl, sec-butyl, tert-butyl; and wherein R⁴ is selected from hydrido, cyano, methyl, ethyl, n-propyl, isopropyl, n-butyl, isobutyl, sec-butyl, tert-butyl, cyclopropyl, methyl, allyl and cyclopropylcarbonyl.

7. Process according to Claim 6 wherein each of R⁵, R⁷ and R⁸ is selected from methyl, ethyl, n-propyl, isopropyl, n-butyl, isobutyl, sec-butyl and tert-butyl; and wherein R⁶ is hydrido.

8. Process according to Claim 7 wherein R³ is selected from

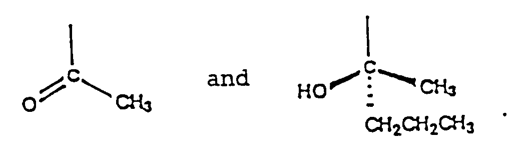

9. Process according to Claim 8 wherein the compound prepared has a double bond between the 17- and 18-position carbon atoms of the carbon-carbon bridge connecting the 6- and 14-position carbon atoms.

10. Process according to Claim 9 wherein the compound prepared is (+)-enantiomer of 7α-(1-(R)-hydroxy-1-methylbutyl)-6,14-endo-ethenotetrahydrooripavine.

11. Process according to Claim 9 wherein the compound prepared is (+)-enantiomer of 7α-(1-(R)-hydroxy-1-methylbutyl)-6,14-endo-ethenotetrahydrothebaine.

12. Process according to Claim 9 wherein the compound prepared is (+)-enantiomer of 7α-acetyl-6,14-endo-ethenotetrahydrothebaine.

13. Process according to Claim 8 wherein the compound prepared has a single bond between the 17- and 18-position carbon atoms of the carbon-carbon bridge connecting the 6- and 14-position carbon atoms.

14. Process according to Claim 13 wherein the compound prepared is selected from the group consisting of (+)-enantiomer of 7α-acetyl-6,14-endo-ethanotetrahydrothebaine; (+)-enantiomer of 7α-(1-hydroxy-1-methylethyl)-6,14-endo-ethanotetrahydrothebaine; (+)-enantiomer of N-cyano-7α-(1-hydroxy-1-methylethyl)-6,14-endo-ethanotetrahydronorthebaine; (+)-enantiomer of 7α-(1-hydroxy-1-methylethyl)-6,14-endo-ethanotetrahydronororipavine; (+)-enantiomer of N-cyclopropylcarbonyl-7α-(1-hydroxy-1-methylethyl)-6,14-endo-ethanotetrahydronororipavine; and (+)-enantiomer of N-cyclopropylmethyl-7α-(1-hydroxy-1-methylethyl)-6,14-endo-ethanotetrahydronororipavine.

15. Process according to Claim 14 wherein the compound prepared is (+)-enantiomer of N-cyclopropylmethyl-7α-(1-(R)-hydroxy-1-methylethyl)-6,14-endo-ethanotetrahydronororipavine.

16. Process according to Claim 13 wherein the compound prepared is group consisting of (+)enantiomer of 7α-(1-hydroxy-1-tert-butyl-ethyl)-6,14-endo-ethanotetrahydrothebaine; (+)-enantiomer of N-cyano-7α-(1-hydroxy-1-tert-butyl-ethyl)-6,14-endo-ethanotetrahydronorthebaine; (+)-enantiomer of 7α-(1-hydroxy-1-methyl-tert-butyl)- and (+)-enantiomer of N-cyclopropylmethyl-7α-(1-hydroxy-1-tert-butyl-ethyl)-6,14-endo-ethanotetrahydronororipavine.

17. Process according to Claim 16 wherein the compound prepared is (+)-enantiomer of N-cyclopropylmethyl-7α-(1-(R)-hydroxy-1-methyl-tert-butyl-ethyl)-6,14-endo-ethanotetrahydronororipavine.

18. A process for preparing a pharmaceutical composition comprising admixing one or more pharmaceutically-acceptable carriers with a substantially non-opiate, epoxymorphinan derivative and one or more pharmaceutically-acceptable excipients, characterized in that said non-opiate epoxymorphinan derivative is provided by one or more compounds of the formula as prepared according to Claim 1.

19. Process according to Claim 18, characterized in that the epoxymorphinan derivative is selected from a group of compounds as prepared according to any of Claims 2-17.

20. Use of a compound as prepared according to Claim 1 for preparing a medicament for suppressing cough in a subject susceptible to or afflicted by cough.

21. Use according to Claim 20 wherein the compound is selected from a group of compounds as prepared according to any of Claims 2-17.

## Claims (Claims for the following Contracting State(s): GR)

1. A compound of the formula 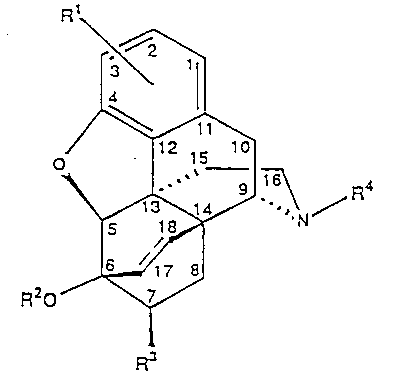 wherein R¹ is selected from hydrido, hydroxy, alkyl, hydroxyalkyl, haloalkyl, cycloalkyl, cycloalkylalkyl, alkoxyalkyl, alkoxy, alkoxycarbonyl, alkylcarboxyl, aryl, aralkyl, aryloxy, arylthio, alkylthio, amino, amido and carboxyl; wherein R² is selected from hydrido, alkyl, hydroxyalkyl, haloalkyl, alkoxyalkyl, haloacyl, aryl and aralkyl; wherein R³ is selected from alkyl, hydroxyalkyl, haloalkyl, cycloalkyl, cycloalkylalkyl, alkoxyalkyl, alkenylalkyl, alkoxy, aryl, aralkyl, aryloxy, alkylthio, amino, amido, carboxyl and 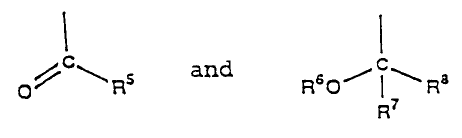 wherein each of R⁵, R⁶, R⁷ and R⁸ is independently selected from hydrido, alkyl, cycloalkyl, phenyl and phenalkyl; wherein R⁴ is selected from hydrido, alkyl, hydroxyalkyl, haloalkyl, cyano, cycloalkyl, cycloalkylalkyl, cycloalkylcarbonyl, alkenylalkyl, alkoxyalkyl, aryl, aralkyl, and carboxyl; wherein any of the foregoing R¹, R², R³ and R⁴ substituents having a substitutable position may be substituted with one or more groups selected from hydroxy, halo, alkyl, cycloalkyl, aryl and aralkyl; and wherein the dashed line between the 17- and 18-position carbon atoms of the carbon-carbon bridge connecting the 6- and 14-position carbon atoms represents a single bond or a double bond; or a pharmaceutically-acceptable salt thereof.

2. Compound of Claim 1 wherein R¹ is selected from hydrido, hydroxy, alkyl, hydroxyalkyl, alkoxyalkyl, alkoxy, alkoxycarbonyl, alkylcarboxyl, phenoxy and phenylthio; wherein R² is selected from hydrido, alkyl, hydroxyalkyl, alkoxyalkyl, phenyl and phenalkyl; wherein R³ is selected from alkyl, hydroxyalkyl, haloalkyl, cycloalkyl, cycloalkylalkyl, cycloalkylcarbonyl, alkenylalkyl, alkoxyalkyl and 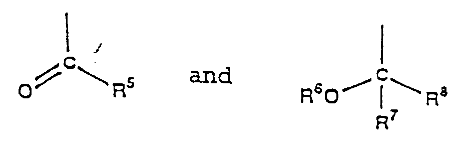 wherein each of R⁵, R⁶, R⁷ and R⁸ is independently selected from hydrido, alkyl, cycloalkyl, phenyl and phenalkyl;
wherein R⁴ is selected from hydrido, alkyl, hydroxyalkyl, haloalkyl, cyano, cycloalkyl, cycloalkylalkyl, cycloalkylcarbonyl, alkenylalkyl, alkoxyalkyl, phenyl and phenalkyl; and wherein any of the foregoing R¹, R², R³ and R⁴ substituents having a substitutable position may be substituted with one or more groups selected from hydroxy, halo, alkyl, cycloalkyl, phenyl and phenalkyl.

3. Compound of Claim 2 wherein R¹ is selected from hydroxy, alkyl, hydroxyalkyl, alkoxyalkyl, alkoxy, alkylcarboxyl and phenoxy; wherein R² is selected from hydrido, alkyl, hydroxyalkyl, alkoxyalkyl, phenyl and phenalkyl; wherein R³ is selected from alkyl, hydroxyalkyl, haloalkyl, cycloalkyl, cycloalkylalkyl, alkoxyalkyl and 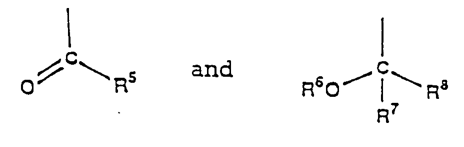 wherein each of R⁵, R⁶, R⁷ and R⁸ is independently selected from hydrido, alkyl, cycloalkyl, phenyl and phenalkyl;
wherein R⁴ is selected from hydrido, alkyl, hydroxyalkyl, cyano, cycloalkyl, cycloalkylalkyl, cycloalkylcarbonyl, allyl, alkoxyalkyl, phenyl and phenalkyl; and wherein any of the foregoing R¹, R², R³ and R⁴ substituents having a substitutable position may be substituted with one or more groups selected from hydroxy, halo, alkyl, cycloalkyl, phenyl and phenalkyl.

4. Compound of Claim 3 wherein R¹ is selected from hydroxy, alkylcarboxyl, alkoxy and phenoxy; wherein R² is selected from hydrido, alkyl, and phenyl; wherein R³ is selected from 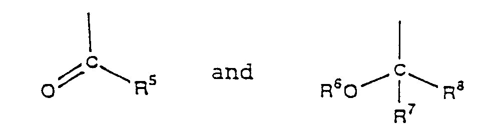 wherein each of R⁵, R⁶, R⁷ and R⁸ is independently selected from hydrido, alkyl, cycloalkyl, phenyl and phenalkyl; wherein R⁴ is selected from hydrido, alkyl, cyano, cycloalkyl, cycloalkylalkyl, cycloalkylcarbonyl, allyl, phenyl, benzyl, phenethyl and phenpropyl; and wherein any of the foregoing R¹, R², R³, R⁴, R⁵, R⁶, R⁷ and R⁸ substituents having a substitutable position may be substituted with one or more groups selected from hydroxy, halo, alkyl and cycloalkyl.

5. Compound of Claim 4 wherein R¹ is selected from hydroxy, alkylcarboxyl, alkoxy and phenoxy; wherein R² is selected from hydrido, alkyl, and phenyl; wherein each of R⁵, R⁶, R⁷ and R⁸ is independently selected from hydrido, alkyl and cycloalkyl; and wherein R⁴ is selected from hydrido, cyano, alkyl, cycloalkyl, cycloalkylalkyl, cycloalkylcarbonyl and allyl.

6. Compound of Claim 5 wherein R¹ is selected from hydroxy, methylcarboxyl, methoxy, ethoxy, n-propoxy, isopropoxy, n-butoxy, isobutoxy, sec-butoxy, tert-butoxy and phenoxy; wherein R² is selected from hydrido, methyl, ethyl, n-propyl, isopropyl, n-butyl, isobutyl, sec-butyl, tert-butyl, acetyl, and phenyl; wherein each of R⁵, R⁶, R⁷ and R⁸ is independently selected from hydrido, methyl, ethyl, n-propyl, isopropyl, n-butyl, isobutyl, sec-butyl, tert-butyl; and wherein R⁴ is selected from hydrido, cyano, methyl, ethyl, n-propyl, isopropyl, n-butyl, isobutyl, sec-butyl, tert-butyl, cyclopropyl, methyl, allyl and cyclopropylcarbonyl.

7. Compound of Claim 6 wherein each of R⁵, R⁷ and R⁸ is selected from methyl, ethyl, n-propyl, isopropyl, n-butyl, isobutyl, sec-butyl and tert-butyl; and wherein R⁶ is hydrido.

8. Compound of Claim 7 wherein R³ is selected from

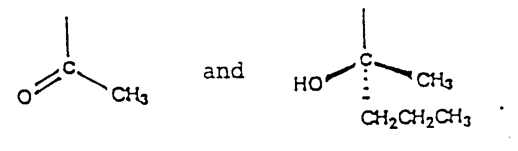

9. Compound of Claim 8 wherein said compound has a double bond between the 17- and 18-position carbon atoms of the carbon-carbon bridge connecting the 6- and 14-position carbon atoms.

10. Compound of Claim 9 which is (+)-enantiomer of 7α-(1-(R)-hydroxy-1-methylbutyl)-6,14-endo-ethenotetrahydrooripavine.

11. Compound of Claim 9 which is (+)-enantiomer of 7α-(1-(R)-hydroxy-1-methylbutyl)-6,14-endo-ethenotetrahydrothebaine.

12. Compound of Claim 9 which is (+)-enantiomer of 7α-acetyl-6,14-endo-ethenotetrahydrothebaine.

13. Compound of Claim 8 wherein said compound has a single bond between the 17- and 18-position carbon atoms of the carbon-carbon bridge connecting the 6- and 14-position carbon atoms.

14. Compound of Claim 13 selected from the group consisting of (+)-enantiomer of 7α-acetyl-6,14-endo-ethanotetrahydrothebaine; (+)-enantiomer of 7α-(1-hydroxy-1-methylethyl)-6,14-endo-ethanotetrahydrothebaine; (+)-enantiomer of N-cyano-7α-(1-hydroxy-1-methylethyl)-6,14-endo-ethanotetrahydronorthebaine; (+)-enantiomer of 7α-(1-hydroxy-1-methylethyl)-6,14-endo-ethanotetrahydronororipavine; (+)-enantiomer of N-cyclopropylcarbonyl-7α-(1-hydroxy-1-methylethyl)-6,14-endo-ethanotetrahydronororipavine; and (+)-enantiomer of N-cyclopropylmethyl-7α-(1-hydroxy-1-methylethyl)-6,14-endo-ethanotetrahydronororipavine.

15. Compound of Claim 14 which is (+)-enantiomer of N-cyclopropylmethyl-7α-(1-(R)-hydroxy-1-methylethyl)-6,14-endo-ethanotetrahydronororipavine.

16. Compound of Claim 13 selected from the group consisting of (+)-enantiomer of 7α-(1-hydroxy-1-tert-butyl-ethyl)-6,14-endo-ethanotetrahydrothebaine; (+)-enantiomer of N-cyano-7α-(1-hydroxy-1-tert-butyl-ethyl)-6,14-endo-ethanotetrahydronorthebaine; and (+)-enantiomer of N-cyclopropylmethyl-7α-(1-hydroxy-1-tert-butyl-ethyl)-6,14-endo-ethanotetrahydronororipavine.

17. Compound of Claim 16 which is (+)-enantiomer of N-cyclopropylmethyl-7α-(1-(R)-hydroxy-1-tert-butyl-ethyl)-6,14-endo-ethanotetrahydronororipavine.

18. A process for preparing a pharmaceutical composition comprising admixing one or more pharmaceutically-acceptable carriers with a substantially non-opiate, epoxymorphinan derivative and one or more pharmaceutically-acceptable excipients, characterized in that said non-opiate epoxymorphinan derivative is provided by one or more compounds of the formula according to Claim 1.

19. Process according to Claim 18, characterized in that the epoxymorphinan derivative is selected from a group of compounds according to any of Claims 2-17.

20. Use of a compound according to Claim 1 for preparing a medicament for suppressing cough in a subject susceptible to or afflicted by cough.

21. Use according to Claim 20 wherein the compound is selected from a group of compounds according to any of Claims 2-17.

## Patentansprüche (Patentansprüche für folgende(n) Vertragsstaat(en): AT, BE, CH, DE, DK, FR, GB, IT, LI, LU, NL, SE)

1. Eine Verbindung der Formel 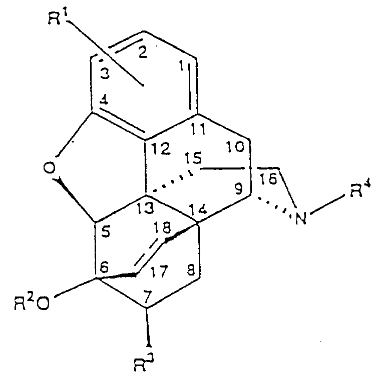 worin R¹ ausgewählt ist aus Hydrido, Hydroxy, Alkyl, Hydroxyalkyl, Halogenalkyl, Cycloalkyl, Cycloalkylalkyl, Alkoxyalkyl, Alkoxy, Alkoxycarbonyl, Alkylcarboxyl, Aryl, Aralkyl, Aryloxy, Arylthio, Alkylthio, Amino, Amido und Carbonyl; R² ausgewählt ist aus Hydrido, Alkyl, Hydroxyalkyl, Halogenalkyl, Alkoxyalkyl, Halogenacyl, Aryl und Aralkyl; R³ ausgewählt ist aus Alkyl, Hydroxyalkyl, Halogenalkyl, Cycloalkyl, Cycloalkylalkyl, Alkoxyalkyl, Alkenylalkyl, Alkoxy, Aryl, Aralkyl, Aryloxy, Alkylthio, Amino, Amido, Carboxyl und 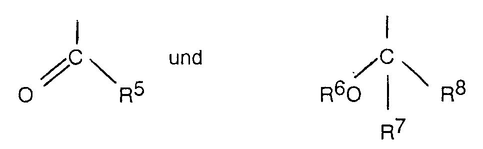 worin jeweils R⁵, R⁶, R⁷ und R⁸ unabhängig voneinander ausgewählt ist aus Hydrido, Alkyl, Cycloalkyl, Phenyl und Phenylalkyl; R⁴ ausgewählt ist aus Hydrido, Alkyl, Hydroxyalkyl, Halogenalkyl, Cyano, Cycloalkyl, Cycloalkylalkyl, Cycloalkylcarbonyl, Alkenylalkyl, Alkoxyalkyl, Aryl, Aralkyl und Carboxyl; worin jeder der vorstehenden R¹, R², R³ und R⁴ Substituenten mit einer substituierbaren Position substituiert sein kann mit einer oder mehreren Gruppen, ausgewählt aus Hydroxy, Halogen, Alkyl, Cycloalkyl, Aryl und Aralkyl; und worin die gestrichelte Linie zwischen den Kohlenstoffatomen der 17- und 18-Position der Kohlenstoff-Kohlenstoff-Brücke, die die Kohlenstoffatome der 6- und 14-Position verbindet, eine Einfachbindung oder eine Doppelbindung darstellt; oder ein pharmazeutisch verträgliches Salz davon.

2. Verbindung nach Anspruch 1, worin R¹ ausgewählt ist aus Hydrido, Hydroxy, Alkyl, Hydroxyalkyl, Alkoxyalkyl, Alkoxy, Alkoxycarbonyl, Alkylcarboxyl, Phenoxy und Phenylthio; R² ausgewählt ist aus Hydrido, Alkyl, Hydroxyalkyl, Alkoxyalkyl, Phenyl und Phenylalkyl; R³ ausgewählt ist aus Alkyl, Hydroxyalkyl, Halogenalkyl, Cycloalkyl, Cycloalkylalkyl, Cycloalkylcarbonyl, Alkenylalkyl, Alkoxyalkyl und 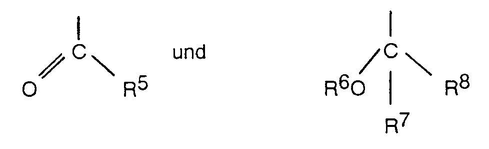 worin jeweils R⁵, R⁶, R⁷ und R⁸ unabhängig voneinander ausgewählt ist aus Hydrido, Alkyl, Cycloalkyl, Phenyl und Phenylalkyl; R⁴ ausgewählt ist aus Hydrido, Alkyl, Hydroxyalkyl, Halogenalkyl, Cyano, Cycloalkyl, Cycloalkylalkyl, Cycloalkylcarbonyl, Alkenylalkyl, Alkoxyalkyl, Phenyl und Phenylalkyl; und worin jeder der vorstehenden R¹, R², R³ und R⁴ Substituenten mit einer substituierbaren Position substituiert sein kann mit einer oder mehreren Gruppen, ausgewählt aus Hydroxy, Halogen, Alkyl, Cycloalkyl, Phenyl und Phenylalkyl.

3. Verbindung nach Anspruch 2, worin R¹ ausgewählt ist aus Hydroxy, Alkyl, Hydroxyalkyl, Alkoxyalkyl, Alkoxy, Alkylcarbonyl und Phenoxy; R² ausgewählt ist aus Hydrido, Alkyl, Hydroxyalkyl, Alkoxyalkyl, Phenyl und Phenylalkyl; R³ ausgewählt ist aus Alkyl, Hydroxyalkyl, Halogenalkyl, Cycloalkyl, Cycloalkylalkyl, Alkoxyalkyl und 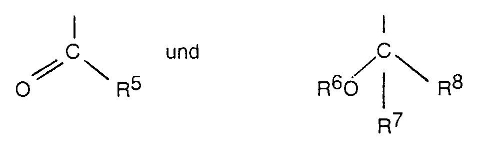 worin jeweils R⁵, R⁶, R⁷ und R⁸ unabhängig voneinander ausgewählt ist aus Hydrido, Alkyl, Cycloalkyl, Phenyl und Phenylalkyl; R⁴ ausgewählt ist aus Hydrido, Alkyl, Hydroxyalkyl, Cyano, Cycloalkyl, Cycloalkylalkyl, Cycloalkylcarbonyl, Allyl, Alkoxyalkyl, Phenyl und Phenylalkyl; und worin jeder der vorstehenden R¹, R², R³ und R⁴ Substituenten mit einer substituierbaren Position substituiert sein kann mit einer oder mehreren Gruppen, ausgewählt aus Hydroxy, Halogen, Alkyl, Cycloalkyl, Phenyl und Phenylalkyl.

4. Verbindung nach Anspruch 3, worin R¹ ausgewählt ist aus Hydroxy, Alkylcarboxyl, Alkoxy und Phenoxy; R² ausgewählt ist aus Hydrido, Alkyl und Phenyl; R³ ausgewählt ist aus 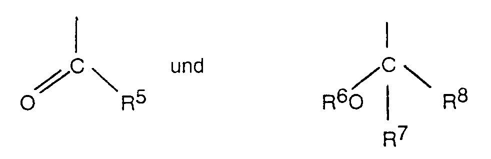 worin jeweils R⁵, R⁶, R⁷ und R⁸ unabhängig voneinander ausgewählt ist aus Hydrido, Alkyl, Cycloalkyl, Phenyl und Phenylalkyl; R⁴ ausgewählt ist aus Hydrido, Alkyl, Cyano, Cycloalkyl, Cycloalkylalkyl, Cycloalkylcarbonyl, Allyl, Phenyl, Benzyl, Phenethyl und Phenpropyl; und worin jeder der vorstehenden R¹, R², R³, R⁴, R⁵, R⁶, R⁷ und R⁸ Substituenten mit einer substituierbaren Position substituiert sein kann mit einer oder mehreren Gruppen, ausgewählt aus Hydroxy, Halogen, Alkyl und Cycloalkyl.

5. Verbindung nach Anspruch 4, worin R¹ ausgewählt ist aus Hydroxy, Alkylcarboxyl, Alkoxy und Phenoxy; R² ausgewählt ist aus Hydrido, Alkyl und Phenyl; jeweils R⁵, R⁶, R⁷ und R⁸ ausgewählt ist aus Hydrido, Alkyl und Cycloalkyl; und R⁴ ausgewählt ist aus Hydrido, Cyano, Alkyl, Cycloalkyl, Cycloalkylalkyl, Cycloalkylcarbonyl und Allyl.

6. Verbindung nach Anspruch 5, worin R¹ ausgewählt ist aus Hydroxy, Methylcarboxyl, Methoxy, Ethoxy, n-Propoxy, Isopropoxy, n-Butoxy, Isobutoxy, sec-Butoxy, tert-Butoxy und Phenoxy; R² ausgewählt ist aus Hydrido, Methyl, Ethyl, n-Propyl, Isopropyl, n-Butyl, Isobutyl, sec-Butyl, tert-Butyl, Acetyl und Phenyl; worin jeweils R⁵, R⁶, R⁷ und R⁸ unabhängig voneinander ausgewählt ist aus Hydrido, Methyl, Ethyl, n-Propyl, Isopropyl, n-Butyl, Isobutyl, sec-Butyl, tert-Butyl; und worin R⁴ ausgewählt ist aus Hydrido, Cyano, Methyl, Ethyl, n-Propyl, Isopropyl, n-Butyl, Isobutyl, sec-Butyl, tert-Butyl, Cyclopropyl, Methyl, Allyl und Cyclopropylcarbonyl.

7. Verbindung nach Anspruch 6, worin jeweils R⁵, R⁷ und R⁸ ausgewählt ist aus Methyl, Ethyl, n-Propyl, Isopropyl, n-Butyl, Isobutyl, sec-Butyl und tert-Butyl, und worin R⁶ Hydrido ist.

8. Verbindung nach Anspruch 7, worin R³ ausgewählt ist aus

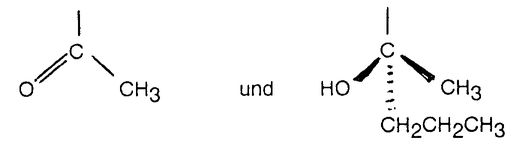

9. Verbindung nach Anspruch 8, worin diese Verbindung eine Doppelbindung zwischen den Kohlenstoffatomen der 17- und 18-Position der Kohlenstoff-Kohlenstoff-Brücke aufweist, die die Kohlenstoffatome der 6- und 14-Position verbindet.

10. Verbindung nach Anspruch 9, nämlich (+)-Enantiomer von 7alpha-(1-(R)-Hydroxy-1-methylbutyl)-6,14-endo-ethenotetrahydrooripavin.

11. Verbindung nach Anspruch 9, nämlich (+)-Enantiomer von 7alpha-(1-(R)-Hydroxy-1-methylbutyl)-6,14-endo-ethenotetrahydrothebain.

12. Verbindung nach Anspruch 9, nämlich (+)-Enantiomer von 7alpha-Acetyl-6,14-endo-ethenotetrahydrothebain.

13. Verbindung nach Anspruch 8, worin diese Verbindung eine Einfachbindung zwischen den Kohlenstoffatomen der 17- und 18-Position der Kohlenstoff-Kohlenstoff-Brücke aufweist, die die Kohlenstoffatome der 6- und 14-Position verbindet.

14. Verbindung nach Anspruch 13, ausgewählt aus der Gruppe bestehend aus (+)-Enantiomer von 7alpha-Acetyl-6,14-endo-ethanotetrahydrothebain, (+)-Enantiomer von 7alpha-(1-Hydroxy-1-methylethyl)-6,14-endo-ethanotetrahydrothebain, (+)-Enantiomer von N-Cyano-7alpha-(1-hydroxy-1-methylethyl)-6,14-endo-ethanotetrahydronorthebain, (+)-Enantiomer von 7alpha-(1-Hydroxy-1-methylethyl)-6,14-endo-ethanotetrahydronororipavin, (+)-Enantiomer von N-Cyclopropylcarbonyl-7alpha-(1-hydroxy-1-methylethyl)-6,14-endo-ethanotetrahydronororipavin und (+)-Enantiomer von N-Cyclopropylmethyl-7alpha-(1-hydroxy-1-methylethyl)-6,14-endo-ethanotetrahydronororipavin.

15. Verbindung nach Anspruch 14, nämlich (+)-Enantiomer von N-Cyclopropylmethyl-7alpha-(1-(R)-hydroxy-1-methylethyl)-6,14-endo-ethanotetrahydronororipavin.

16. Verbindung nach Anspruch 13 ausgewählt aus der Gruppe bestehend aus (+)-Enantiomer von 7alpha-(1-Hydroxy-1-tert-butyl-ethyl)-6,14-endo-ethanotetrahydrothebain, (+)-Enantiomer von N-Cyano-7alpha-(1-hydroxy-1-tert-butyl-ethyl)-6,14-endo-ethanotetrahydronorthebain, und (+)-Enantiomer von N-Cyclopropylmethyl-7alpha-(1-hydroxy-1-tert-butyl-ethyl)-6,14-endo-ethanotetrahydronororipavin.

17. Verbindung nach Anspruch 16, nämlich (+)-Enantiomer von N-Cyclopropylmethyl-7alpha-(1-(R)-hydroxy-1-tert-butyl-ethyl)-6,14-endo-ethanotetrahydronororipavin.

18. Eine pharmazeutische Zusammensetzung, enthaltend ein im wesentlichen nicht opiates Epoxymorphinanderivat und einen oder mehrere pharmazeutisch verträgliche Exzipientien, wobei das nicht opiate Epoxymorphinanderivat bereitgestellt wird durch eine oder mehrere Verbindungen der Formel gemäß Anspruch 1.

19. Eine pharmazeutische Zusammensetzung gemäß Anspruch 18, worin das nicht opiate Epoxymorphinanderivat ausgewählt ist aus einer Verbindung gemäß einem der Ansprüche 2-17.

20. Verwendung einer Verbindung gemäß Anspruch 1 zur Herstellung eines Medikamentes zum Stillen von Husten in einem Subjekt, das gegenüber Husten empfänglich ist oder darunter leidet.

21. Verwendung nach Anspruch 20, worin die Verbindung ausgewählt ist aus einer Verbindung gemäß einem der Ansprüche 2 bis 17.

## Patentansprüche (Patentansprüche für folgende(n) Vertragsstaat(en): ES)

1. Ein Verfahren zur Herstellung einer Verbindung der Formel 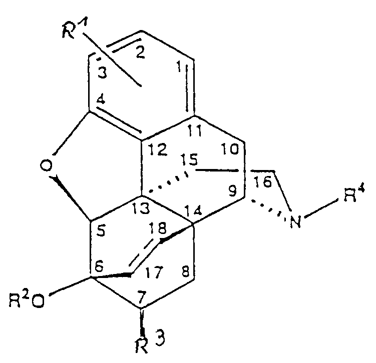 worin R¹ ausgewählt ist aus Hydrido, Hydroxy, Alkyl, Hydroxyalkyl, Halogenalkyl, Cycloalkyl, Cycloalkylalkyl, Alkoxyalkyl, Alkoxy, Alkoxycarbonyl, Alkylcarboxyl, Aryl, Aralkyl, Aryloxy, Arylthio, Alkylthio, Amino, Amido und Carbonyl; R² ausgewählt ist aus Hydrido, Alkyl, Hydroxyalkyl, Halogenalkyl, Alkoxyalkyl, Halogenacyl, Aryl und Aralkyl; R³ ausgewählt ist aus Alkyl, Hydroxyalkyl, Halogenalkyl, Cycloalkyl, Cycloalkylalkyl, Alkoxyalkyl, Alkenylalkyl, Alkoxy, Aryl, Aralkyl, Aryloxy, Alkylthio, Amino, Amido, Carboxyl und 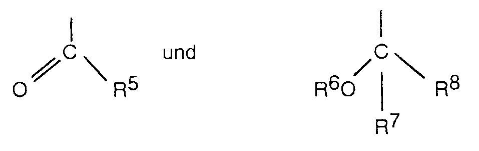 worin jeweils R⁵, R⁶, R⁷ und R⁸ unabhängig voneinander ausgewählt ist aus Hydrido, Alkyl, Cycloalkyl, Phenyl und Phenylalkyl; R⁴ ausgewählt ist aus Hydrido, Alkyl, Hydroxyalkyl, Halogenalkyl, Cyano, Cycloalkyl, Cycloalkylalkyl, Cycloalkylcarbonyl, Alkenylalkyl, Alkoxyalkyl, Aryl, Aralkyl und Carboxyl; worin jeder der vorstehenden R¹, R², R³ und R⁴ Substituenten mit einer substituierbaren Position substituiert sein kann mit einer oder mehreren Gruppen, ausgewählt aus Hydroxy, Halogen, Alkyl, Cycloalkyl, Aryl und Aralkyl; und worin die gestrichelte Linie zwischen den Kohlenstoffatomen der 17- und 18-Position der Kohlenstoff-Kohlenstoff-Brücke, die die Kohlenstoffatome der 6- und 14-Position verbindet, eine Einfachbindung oder eine Doppelbindung darstellt; oder eines pharmazeutisch verträglichen Salzes davon, dadurch gekennzeichnet, daß man eine Verbindung der Formel (a) 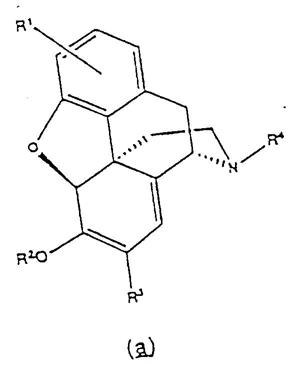 worin R¹ bis R⁴ vorstehende Bedeutung haben, unter geeigneten Bedingungen mit einem Vinylketon der Formel (b) 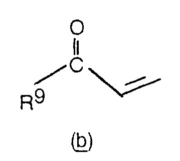 umsetzt, worin R⁹ ein Vorläufer-Rest ist, der dann im R³-Substituenten des Endproduktes enthalten ist, und somit zur Verbindung (c) der Formel 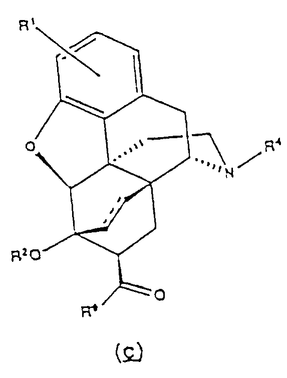 führt, worin alle Substituenten vorstehende Bedeutung haben,
diese Verbindung (c) dann unter Anwendung eines Grignard-Reagenzes (d) 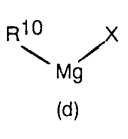 umsetzt, worin X ein Halogenid und R¹⁰ einen Vorläufer-Rest darstellen, zur Einfügung in den R³-Substituenten des Endproduktes, und somit zu den gewünschten Endprodukten führt, die gegebenenfalls auf bekannte Weise in ihre entsprechenden pharmazeutisch-verträglichen Salze überführt werden können.

2. Verfahren gemäß Anspruch 1, worin R¹ der hergestellten Verbindung ausgewählt ist aus Hydrido, Hydroxy, Alkyl, Hydroxyalkyl, Alkoxyalkyl, Alkoxy, Alkoxycarbonyl, Alkylcarboxyl, Phenoxy und Phenylthio; R² ausgewählt ist aus Hydrido, Alkyl, Hydroxyalkyl, Alkoxyalkyl, Phenyl und Phenylalkyl; R³ ausgewählt ist aus Alkyl, Hydroxyalkyl, Halogenalkyl, Cycloalkyl, Cycloalkylalkyl, Cycloalkylcarbonyl, Alkenylalkyl, Alkoxyalkyl und 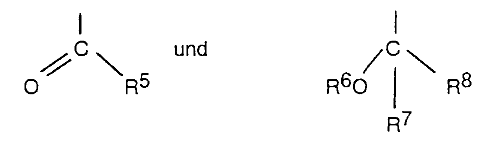 worin jeweils R⁵, R⁶, R⁷ und R⁸ unabhängig voneinander ausgewählt ist aus Hydrido, Alkyl, Cycloalkyl, Phenyl und Phenylalkyl; R⁴ ausgewählt ist aus Hydrido, Alkyl, Hydroxyalkyl, Halogenalkyl, Cyano, Cycloalkyl, Cycloalkylalkyl, Cycloalkylcarbonyl, Alkenylalkyl, Alkoxyalkyl, Phenyl und Phenylalkyl; und worin jeder der vorstehenden R¹, R², R³ und R⁴ Substituenten mit einer substituierbaren Position substituiert sein kann mit einer oder mehreren Gruppen, ausgewählt aus Hydroxy, Halogen, Alkyl, Cycloalkyl, Phenyl und Phenylalkyl.

3. Verfahren gemäß Anspruch 2, worin R¹ der hergestellten Verbindung ausgewählt ist aus Hydroxy, Alkyl, Hydroxyalkyl, Alkoxyalkyl, Alkoxy, Alkylcarbonyl und Phenoxy; R² ausgewählt ist aus Hydrido, Alkyl, Hydroxyalkyl, Alkoxyalkyl, Phenyl und Phenylalkyl; R³ ausgewählt ist aus Alkyl, Hydroxyalkyl, Halogenalkyl, Cycloalkyl, Cycloalkylalkyl, Alkoxyalkyl und 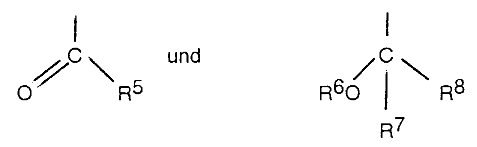 worin jeweils R⁵, R⁶, R⁷ und R⁶ unabhängig voneinander ausgewählt ist aus Hydrido, Alkyl, Cycloalkyl, Phenyl und Phenylalkyl; R⁴ ausgewählt ist aus Hydrido, Alkyl, Hydroxyalkyl, Cyano, Cycloalkyl, Cycloalkylalkyl, Cycloalkylcarbonyl, Allyl, Alkoxyalkyl, Phenyl und Phenylalkyl; und worin jeder der vorstehenden R¹, R², R³ und R⁴ Substituenten mit einer substituierbaren Position substituiert sein kann mit einer oder mehreren Gruppen, ausgewählt aus Hydroxy, Halogen, Alkyl, Cycloalkyl, Phenyl und Phenylalkyl.

4. Verfahren nach Anspruch 3, worin R¹ der hergestellten Verbindung ausgewählt ist aus Hydroxy, Alkylcarboxyl, Alkoxy und Phenoxy; R² ausgewählt ist aus Hydrido, Alkyl und Phenyl; R³ ausgewählt ist aus 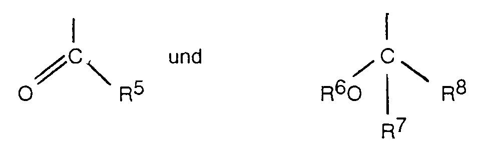 worin jeweils R⁵, R⁶, R⁷ und R⁸ unabhängig voneinander ausgewählt ist aus Hydrido, Alkyl, Cycloalkyl, Phenyl und Phenylalkyl; R⁴ ausgewählt ist aus Hydrido, Alkyl, Cyano, Cycloalkyl, Cycloalkylalkyl, Cycloalkylcarbonyl, Allyl, Phenyl, Benzyl, Phenethyl und Phenpropyl; und worin jeder der vorstehenden R¹, R², R³, R⁴, R⁵, R⁶, R⁷ und R⁸ Substituenten mit einer substituierbaren Position substituiert sein kann mit einer oder mehreren Gruppen, ausgewählt aus Hydroxy, Halogen, Alkyl und Cycloalkyl.

5. Verfahren gemäß Anspruch 4, worin R¹ der hergestellten Verbindung ausgewählt ist aus Hydroxy, Alkylcarboxyl, Alkoxy und Phenoxy; R² ausgewählt ist aus Hydrido, Alkyl und Phenyl; jeweils R⁵, R⁶, R⁷ und R⁸ ausgewählt ist aus Hydrido, Alkyl und Cycloalkyl; und R⁴ ausgewählt ist aus Hydrido, Cyano, Alkyl, Cycloalkyl, Cycloalkylalkyl, Cycloalkylcarbonyl und Allyl.

6. Verfahren gemäß Anspruch 4, worin R¹ der hergestellten Verbindung ausgewählt ist aus Hydroxy, Methylcarboxyl, Methoxy, Ethoxy, n-Propoxy, Isopropoxy, n-Butoxy, Isobutoxy, sec-Butoxy, tert-Butoxy und Phenoxy; R² ausgewählt ist aus Hydrido, Methyl, Ethyl, n-Propyl, Isopropyl, n-Butyl, Isobutyl, sec-Butyl, tert-Butyl, Acetyl und Phenyl; worin jeweils R⁵, R⁶, R⁷ und R⁸ unabhängig voneinander ausgewählt ist aus Hydrido, Methyl, Ethyl, n-Propyl, Isopropyl, n-Butyl, Isobutyl, sec-Butyl, tert-Butyl; und worin R⁴ ausgewählt ist aus Hydrido, Cyano, Methyl, Ethyl, n-Propyl, Isopropyl, n-Butyl, Isobutyl, sec-Butyl, tert-Butyl, Cyclopropyl, Methyl, Allyl und Cyclopropylcarbonyl.

7. Verfahren nach Anspruch 6, worin jeweils R⁵, R⁷ und R⁸ ausgewählt ist aus Methyl, Ethyl, n-Propyl, Isopropyl, n-Butyl, Isobutyl, sec-Butyl und tert-Butyl, und worin R⁶ Hydrido ist.

8. Verfahren nach Anspruch 7, worin R³ ausgewählt ist aus

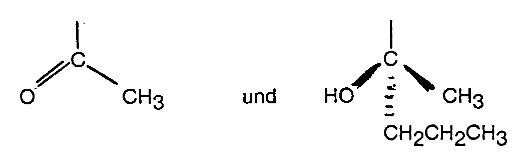

9. Verfahren nach Anspruch 8, worin die hergestellte Verbindung eine Doppelbindung zwischen den Kohlenstoffatomen der 17- und 18-Position der Kohlenstoff-Kohlenstoff-Brücke aufweist, die die Kohlenstoffatome der 6-und 14-Position verbindet.

10. Verfahren nach Anspruch 9, worin die hergestellte Verbindung (+)-Enantiomer von 7alpha-(1-(R)-Hydroxy-1-methylbutyl)-6,14-endo-ethenotetrahydrooripavin ist.

11. Verfahren nach Anspruch 9, worin die hergestellte Verbindung (+)-Enantiomer von 7alpha-(1-(R)-Hydroxy-1-methylbutyl)-6,14-endo-ethenotetrahydrothebain ist.

12. Verfahren nach Anspruch 9, worin die hergestellte Verbindung (+)-Enantiomer von 7alpha-Acetyl-6,14-endo-ethenotetrahydrothebain ist.

13. Verfahren nach Anspruch 8, worin die hergestellte Verbindung eine Einfachbindung zwischen den Kohlenstoffatomen der 17- und 18-Position der Kohlenstoff-Kohlenstoff-Brücke aufweist, die die Kohlenstoffatome der 6-und 14-Position verbindet.

14. Verfahren nach Anspruch 13, worin die hergestellte Verbindung ausgewählt ist aus der Gruppe bestehend aus (+)-Enantiomer von 7alpha-Acetyl-6,14-endo-ethanotetrahydrothebain, (+)-Enantiomer von 7alpha-(1-Hydroxy-1-methylethyl)-6,14-endo-ethanotetrahydrothebain, (+)-Enantiomer von N-Cyano-7alpha-(1-hydroxy-1-methylethyl)-6,14-endo-ethano-tetrahydronorthebain, (+)-Enantiomer von 7alpha-(1-Hydroxy-1-methylethyl)-6,14-endo-ethanotetrahydronororipavin, (+)-Enantiomer von N-Cyclopropylcarbonyl-7alpha-(1-hydroxy-1-methylethyl)-6,14-endo-ethanotetrahydronororipavin und (+)-Enantiomer von N-Cyclopropylmethyl-7alpha-(1-hydroxy-1-methylethyl)-6,14-endo-ethanotetrahydronororipavin.

15. Verfahren nach Anspruch 14, worin die hergestellte Verbindung (+)-Enantiomer von N-Cyclo-propylmethyl-7alpha-(1-(R)-hydroxy-1-methylethyl)-6,14-endo-ethanotetrahydronororipavin ist.

16. Verfahren nach Anspruch 13, worin die hergestellte Verbindung ausgewählt ist aus der Gruppe bestehend aus (+)-Enantiomer von 7alpha-(1-Hydroxy-1-tert-butyl-ethyl)-6,14-endo-ethanotetrahydrothebain, (+)-Enantiomer von N-Cyano-7alpha-(1-hydroxy-1-tert-butyl-ethyl)-6,14-endo-ethanotetrahydronorthebain, und (+)-Enantiomer von N-Cyclopropylmethyl-7alpha-(1-hydroxy-1-tert-butyl-ethyl)-6,14-endo-ethanotetrahydronororipavin.

17. Verfahren nach Anspruch 16, worin die hergestellte Verbindung (+)-Enantiomer von N-Cyclopropyl-methyl-7alpha-(1-(R)-hydroxy-1-tert-butyl-ethyl)-6,14-endo-ethanotetrahydronororipavin ist.

18. Ein Verfahren zur Herstellung einer pharmazeutischen Zusammensetzung, umfassend das Vermischen von einem oder mehreren pharmazeutisch verträglichen Trägern mit einem im wesentlichen nicht opiaten Epoxymorphinanderivat und einem oder mehreren pharmazeutisch verträglichen Exzipientien, dadurch gekennzeichnet, daß das nicht opiate Epoxymorphinanderivat bereitgestellt wird durch eine oder mehrere Verbindungen der Formel, wie sie nach Anspruch 1 hergestellt wurden.

19. Verfahren nach Anspruch 18, dadurch gekennzeichnet, daß das Epoxymorphinanderivat ausgewählt ist aus einer Gruppe von Verbindungen, wie sie gemäß einem der Ansprüche 2-17 hergestellt wurden.

20. Verwendung einer Verbindung, wie sie nach Anspruch 1 hergestellt wurde, zur Herstellung eines Medikamentes zum Stillen von Husten in einem Subjekt, das gegenüber Husten empfänglich ist oder darunter leidet.

21. Verwendung nach Anspruch 20, worin die Verbindung ausgewählt ist aus einer Gruppe von Verbindungen, wie sie gemäß einem der Ansprüche 2-17 hergestellt wurden.

## Patentansprüche (Patentansprüche für folgende(n) Vertragsstaat(en): GR)

1. Eine Verbindung der Formel 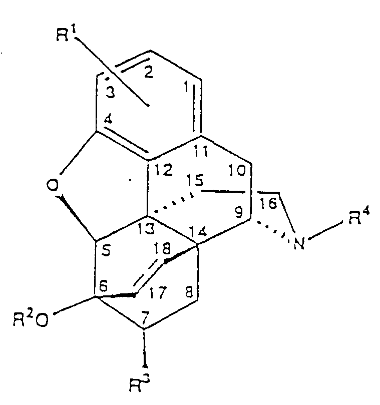 worin R¹ ausgewählt ist aus Hydrido, Hydroxy, Alkyl, Hydroxyalkyl, Halogenalkyl, Cycloalkyl, Cycloalkylalkyl, Alkoxyalkyl, Alkoxy, Alkoxycarbonyl, Alkylcarboxyl, Aryl, Aralkyl, Aryloxy, Arylthio, Alkylthio, Amino, Amido und Carbonyl; R² ausgewählt ist aus Hydrido, Alkyl, Hydroxyalkyl, Halogenalkyl, Alkoxyalkyl, Halogenacyl, Aryl und Aralkyl; R³ ausgewählt ist aus Alkyl, Hydroxyalkyl, Halogenalkyl, Cycloalkyl, Cycloalkylalkyl, Alkoxyalkyl, Alkenylalkyl, Alkoxy, Aryl, Aralkyl, Aryloxy, Alkylthio, Amino, Amido, Carboxyl und 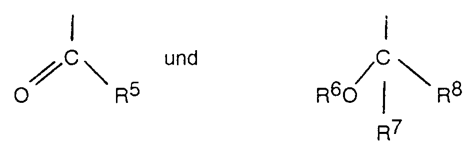 worin jeweils R⁵, R⁶, R⁷ und R⁸ unabhängig voneinander ausgewählt ist aus Hydrido, Alkyl, Cycloalkyl, Phenyl und Phenylalkyl; R⁴ ausgewählt ist aus Hydrido, Alkyl, Hydroxyalkyl, Halogenalkyl, Cyano, Cycloalkyl, Cycloalkylalkyl, Cycloalkylcarbonyl, Alkenylalkyl, Alkoxyalkyl, Aryl, Aralkyl und Carboxyl; worin jeder der vorstehenden R¹, R², R³ und R⁴ Substituenten mit einer substituierbaren Position substituiert sein kann mit einer oder mehreren Gruppen, ausgewählt aus Hydroxy, Halogen, Alkyl, Cycloalkyl, Aryl und Aralkyl; und worin die gestrichelte Linie zwischen den Kohlenstoffatomen der 17- und 18-Position der Kohlenstoff-Kohlenstoff-Brücke, die die Kohlenstoffatome der 6- und 14-Position verbindet, eine Einfachbindung oder eine Doppelbindung darstellt; oder ein pharmazeutisch verträgliches Salz davon.

2. Verbindung nach Anspruch 1, worin R¹ ausgewählt ist aus Hydrido, Hydroxy, Alkyl, Hydroxyalkyl, Alkoxyalkyl, Alkoxy, Alkoxycarbonyl, Alkylcarboxyl, Phenoxy und Phenylthio; R² ausgewählt ist aus Hydrido, Alkyl, Hydroxyalkyl, Alkoxyalkyl, Phenyl und Phenylalkyl; R³ ausgewählt ist aus Alkyl, Hydroxyalkyl, Halogenalkyl, Cycloalkyl, Cycloalkylalkyl, Cycloalkylcarbonyl, Alkenylalkyl, Alkoxyalkyl und 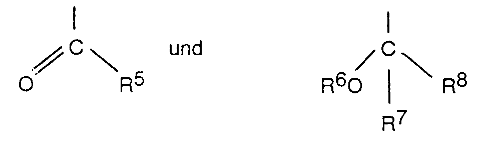 worin jeweils R⁵, R⁶, R⁷ und R⁸ unabhängig voneinander ausgewählt ist aus Hydrido, Alkyl, Cycloalkyl, Phenyl und Phenylalkyl; R⁴ ausgewählt ist aus Hydrido, Alkyl, Hydroxyalkyl, Halogenalkyl, Cyano, Cycloalkyl, Cycloalkylalkyl, Cycloalkylcarbonyl, Alkenylalkyl, Alkoxyalkyl, Phenyl und Phenylalkyl; und worin jeder der vorstehenden R¹, R², R³ und R⁴ Substituenten mit einer substituierbaren Position substituiert sein kann mit einer oder mehreren Gruppen, ausgewählt aus Hydroxy, Halogen, Alkyl, Cycloalkyl, Phenyl und Phenylalkyl.

3. Verbindung nach Anspruch 2, worin R¹ ausgewählt ist aus Hydroxy, Alkyl, Hydroxyalkyl, Alkoxyalkyl, Alkoxy, Alkylcarbonyl und Phenoxy; R² ausgewählt ist aus Hydrido, Alkyl, Hydroxyalkyl, Alkoxyalkyl, Phenyl und Phenylalkyl; R³ ausgewählt ist aus Alkyl, Hydroxyalkyl, Halogenalkyl, Cycloalkyl, Cycloalkylalkyl, Alkoxyalkyl und 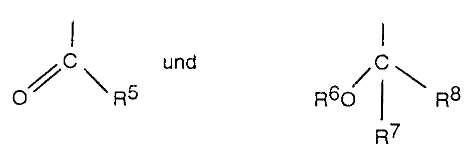 worin jeweils R⁵, R⁶, R⁷ und R⁸ unabhängig voneinander ausgewählt ist aus Hydrido, Alkyl, Cycloalkyl, Phenyl und Phenylalkyl; R⁴ ausgewählt ist aus Hydrido, Alkyl, Hydroxyalkyl, Cyano, Cycloalkyl, Cycloalkylalkyl, Cycloalkylcarbonyl, Allyl, Alkoxyalkyl, Phenyl und Phenylalkyl; und worin jeder der vorstehenden R¹, R², R³ und R⁴ Substituenten mit einer substituierbaren Position substituiert sein kann mit einer oder mehreren Gruppen, ausgewählt aus Hydroxy, Halogen, Alkyl, Cycloalkyl, Phenyl und Phenylalkyl.

4. Verbindung nach Anspruch 3, worin R¹ ausgewählt ist aus Hydroxy, Alkylcarboxyl, Alkoxy und Phenoxy; R² ausgewählt ist aus Hydrido, Alkyl und Phenyl; R³ ausgewählt ist aus 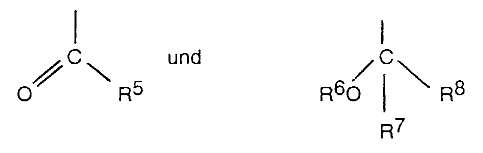 worin jeweils R⁵, R⁶, R⁷ und R⁸ unabhängig voneinander ausgewählt ist aus Hydrido, Alkyl, Cycloalkyl, Phenyl und Phenylalkyl; R⁴ ausgewählt ist aus Hydrido, Alkyl, Cyano, Cycloalkyl, Cycloalkylalkyl, Cycloalkylcarbonyl, Allyl, Phenyl, Benzyl, Phenethyl und Phenpropyl; und worin jeder der vorstehenden R¹, R², R³, R⁴, R⁵, R⁶, R⁷ und R⁸ Substituenten mit einer substituierbaren Position substituiert sein kann mit einer oder mehreren Gruppen, ausgewählt aus Hydroxy, Halogen, Alkyl und Cycloalkyl.

5. Verbindung nach Anspruch 4, worin R¹ ausgewählt ist aus Hydroxy, Alkylcarboxyl, Alkoxy und Phenoxy; R² ausgewählt ist aus Hydrido, Alkyl und Phenyl; jeweils R⁵, R⁶, R⁷ und R⁸ ausgewählt ist aus Hydrido, Alkyl und Cycloalkyl; und R⁴ ausgewählt ist aus Hydrido, Cyano, Alkyl, Cycloalkyl, Cycloalkylalkyl, Cycloalkylcarbonyl und Allyl.

6. Verbindung nach Anspruch 5, worin R¹ ausgewählt ist aus Hydroxy, Methylcarboxyl, Methoxy, Ethoxy, n-Propoxy, Isopropoxy, n-Butoxy, Isobutoxy, sec-Butoxy, tert-Butoxy und Phenoxy; R² ausgewählt ist aus Hydrido, Methyl, Ethyl, n-Propyl, Isopropyl, n-Butyl, Isobutyl, sec-Butyl, tert-Butyl, Acetyl und Phenyl; worin jeweils R⁵, R⁶, R⁷ und R⁸ unabhängig voneinander ausgewählt ist aus Hydrido, Methyl, Ethyl, n-Propyl, Isopropyl, n-Butyl, Isobutyl, sec-Butyl, tert-Butyl; und worin R⁴ ausgewählt ist aus Hydrido, Cyano, Methyl, Ethyl, n-Propyl, Isopropyl, n-Butyl, Isobutyl, sec-Butyl, tert-Butyl, Cyclopropyl, Methyl, Allyl und Cyclopropylcarbonyl.

7. Verbindung nach Anspruch 6, worin jeweils R⁵, R⁷ und R⁸ ausgewählt ist aus Methyl, Ethyl, n-Propyl, Isopropyl, n-Butyl, Isobutyl, sec-Butyl und tert-Butyl, und worin R⁶ Hydrido ist.

8. Verbindung nach Anspruch 7, worin R³ ausgewählt ist aus

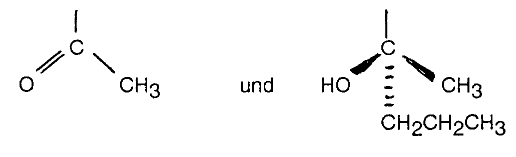

9. Verbindung nach Anspruch 8, worin diese Verbindung eine Doppelbindung zwischen den Kohlenstoffatomen der 17- und 18-Position der Kohlenstoff-Kohlenstoff-Brücke aufweist, die die Kohlenstoffatome der 6- und 14-Position verbindet.

10. Verbindung nach Anspruch 9, nämlich (+)-Enantiomer von 7alpha-(1-(R)-Hydroxy-1-methylbutyl)-6,14-endo-ethenotetrahydrooripavin.

11. Verbindung nach Anspruch 9, nämlich (+)-Enantiomer von 7alpha-(1-(R)-Hydroxy-1-methylbutyl)-6,14-endo-ethenotetrahydrothebain.

12. Verbindung nach Anspruch 9, nämlich (+)-Enantiomer von 7alpha-Acetyl-6,14-endo-ethenotetrahydrothebain.

13. Verbindung nach Anspruch 8, worin diese Verbindung eine Einfachbindung zwischen den Kohlenstoffatomen der 17- und 18-Position der Kohlenstoff-Kohlenstoff-Brücke aufweist, die die Kohlenstoffatome der 6- und 14-Position verbindet.

14. Verbindung nach Anspruch 13, ausgewählt aus der Gruppe bestehend aus (+)-Enantiomer von 7alpha-Acetyl-6,14-endo-ethanotetrahydrothebain, (+)-Enantiomer von 7alpha-(1-Hydroxy-1-methylethyl)-6,14-endo-ethanotetrahydrothebain, (+)-Enantiomer von N-Cyano-7alpha-(1-hydroxy-1-methylethyl)-6,14-endo-ethanotetrahydronorthebain, (+)-Enantiomer von 7alpha-(1-Hydroxy-1-methylethyl)-6,14-endo-ethanotetrahydronororipavin, (+)-Enantiomer von N-Cyclopropylcarbonyl-7alpha-(1-hydroxy-1-methylethyl)-6,14-endo-ethanotetrahydronororipavin und (+)-Enantiomer von N-Cyclopropylmethyl-7alpha-(1-hydroxy-1-methylethyl)-6,14-endo-ethanotetrahydronororipavin.

15. Verbindung nach Anspruch 14, nämlich (+)-Enantiomer von N-Cyclopropylmethyl-7alpha-(1-(R)-hydroxy-1-methylethyl)-6,14-endo-ethanotetrahydronororipavin.

16. Verbindung nach Anspruch 13 ausgewählt aus der Gruppe bestehend aus (+)-Enantiomer von 7alpha-(1-Hydroxy-1-tert-butyl-ethyl)-6,14-endo-ethanotetrahydrothebain, (+)-Enantiomer von N-Cyano-7alpha-(1-hydroxy-1-tert-butyl-ethyl)-6,14-endo-ethanotetrahydronorthebain, und (+)-Enantiomer von N-Cyclopropylmethyl-7alpha-(1-hydroxy-1-tert-butyl-ethyl)-6,14-endo-ethanotetrahydronororipavin.

17. Verbindung nach Anspruch 16, nämlich (+)-Enantiomer von N-Cyclopropylmethyl-7alpha-(1-(R)-hydroxy-1-tert-butyl-ethyl)-6,14-endo-ethanotetrahydronororipavin.

18. Ein Verfahren zur Herstellung einer pharmazeutischen Zusammensetzung, umfassend das Vermischen von einem oder mehreren pharmazeutisch verträglichen Trägern mit einem im wesentlichen nicht opiaten Epoxymorphinanderivat und einem oder mehreren pharmazeutisch verträglichen Exzipientien, dadurch gekennzeichnet, daß das nicht opiate Epoxymorphinanderivat bereitgestellt wird durch eine oder mehrere Verbindungen der Formel gemäß Anspruch 1.

19. Verfahren nach Anspruch 18, dadurch gekennzeichnet, daß das Epoxymorphinanderivat ausgewählt ist aus einer Gruppe von Verbindungen gemäß einem der Ansprüche 2 bis 17.

20. Verwendung einer Verbindung, wie sie nach Anspruch 1 hergestellt wurde, zur Herstellung eines Medikamentes zum Stillen von Husten in einem Subjekt, das gegenüber Husten empfänglich ist oder darunter leidet.

21. Verwendung nach Anspruch 20, worin die Verbindung ausgewählt ist aus einer Gruppe von Verbindungen, wie sie gemäß einem der Ansprüche 2-17 hergestellt wurden.

## Revendications (Revendications pour l'(les) Etat(s) contractant(s) suivant(s): AT, BE, CH, DE, DK, FR, GB, IT, LI, LU, NL, SE)

1. Composé de formule 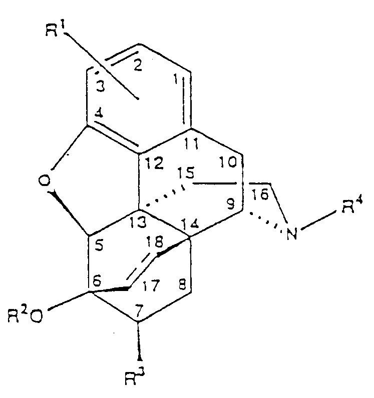 dans laquelle R¹ représente un atome d'hydrogène ou un groupe hydroxyle, alkyle, hydroxyalkyle, halogénoalkyle, cycloalkyle, cycloalkylalkyle, alcoxyalkyle, alcoxy, alcoxycarbonyle, alkylcarboxyle, aryle, aralkyle, aryloxy, arylthio, alkylthio, amino, amido ou carboxyle ; R² représente un atome d'hydrogène ou un groupe alkyle, hydroxyalkyle, halogénoalkyle, alcoxyalkyle, halogénoacyle, aryle ou aralkyle ; R³ représente un groupe alkyle, hydroxyalkyle, halogénoalkyle, cycloalkyle, cycloalkylalkyle, alcoxyalkyle, alcénylalkyle, alcoxy, aryle, aralkyle, aryloxy, alkylthio, amino, amido, carboxyle ou un groupe répondant à l'une des formules 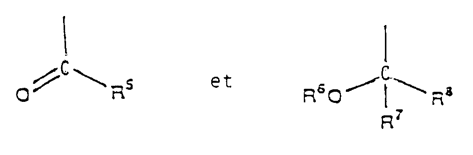 dans lesquelles R⁵, R⁶, R⁷ et R⁸ représentent chacun indépendamment un atome d'hydrogène ou un groupe alkyle, cycloalkyle, phényle ou phénylalkyle ; R⁴ représente un atome d'hydrogène ou un groupe alkyle, hydroxyalkyle, halogénoalkyle, cyano, cycloalkyle, cycloalkylalkyle, cycloalkylcarbonyle, alcénylalkyle, alcoxyalkyle, aryle, aralkyle ou carboxyle ; dans laquelle l'un quelconque des substituants R¹, R², R³ et R⁴ précédents ayant une position substituable peut être substitué par un ou plusieurs substituants choisis parmi les halogènes et les groupes hydroxyle, alkyle, cycloalkyle, aryle et aralkyle ; et dans laquelle le trait interrompu entre les atomes de carbone en positions 17 et 18 du pont carbone-carbone reliant les atomes de carbone en positions 6 et 14 représente une liaison simple ou une liaison double ; ou un de ses sels acceptables en pharmacie.

2. Composé selon la revendication 1, dans lequel R¹ représente un atome d'hydrogène ou un groupe hydroxyle, alkyle, hydroxyalkyle, alcoxyalkyle, alcoxy, alcoxycarbonyle, alkylcarboxyle, phénoxy ou phénylthio ; R² représente un atome d'hydrogène ou un groupe alkyle, hydroxyalkyle, alcoxyalkyle, phényle ou phénylalkyle; R³ représente un groupe alkyle, hydroxyalkyle, halogénoalkyle, cycloalkyle, cycloalkylalkyle, cycloalkylcarbonyle, alcénylcarbonyle, alcoxyalkyle ou un groupe répondant à l'une des formules 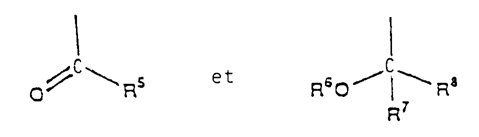 dans lesquelles R⁵, R⁶, R⁷ et R⁸ représentent chacun indépendamment un atome d'hydrogène ou un groupe alkyle, cycloalkyle, phényle ou phénylalkyle ; R⁴ représente un atome d'hydrogène ou un groupe alkyle, hydroxyalkyle, halogénoalkyle, cyano, cycloalkyle, cycloalkylalkyle, cycloalkylcarbonyle, alcénylalkyle, alcoxyalkyle, phényle ou phénylalkyle ; et dans laquelle l'un quelconque des substituants R¹, R², R³ et R⁴ précédents ayant une position substituable peut être substitué par un ou plusieurs substituants choisis parmi les halogènes et les groupes hydroxyle, alkyle, cycloalkyle, phényle et phénylalkyle.

3. Composé selon la revendication 2, dans lequel R¹ représente un groupe hydroxyle, alkyle, hydroxyalkyle, alcoxyalkyle, alcoxy, alkylcarboxyle ou phénoxy ; R² représente un atome d'hydrogène ou un groupe alkyle, hydroxyalkyle, alcoxyalkyle, phényle ou phénylalkyle ; R³ représente un groupe alkyle, hydroxyalkyle, halogénoalkyle, cycloalkyle, cycloalkylalkyle, alcoxyalkyle ou un groupe répondant à l'une des formules 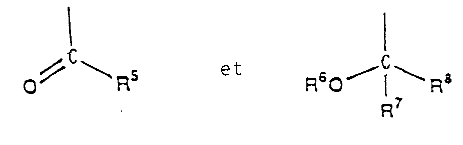 dans lesquelles R⁵, R⁶, R⁷ et R⁸ représentent chacun indépendamment un atome d'hydrogène ou un groupe alkyle, cycloalkyle, phényle ou phénylalkyle ; R⁴ représente un atome d'hydrogène ou un groupe alkyle, cyano, cycloalkyle, cycloalkylalkyle, cycloalkylcarbonyle, allyle, alcoxyalkyle, phényle ou phénylalkyle ; et dans laquelle l'un quelconque des substituants R¹, R², R³ et R⁴ précédents ayant une position substituable peut être substitué par un ou plusieurs substituants choisis parmi les halogènes et les groupes hydroxyle, alkyle, cycloalkyle, phényle et phénylalkyle.

4. Composé selon la revendication 3, dans lequel R¹ représente un groupe hydroxyle, alkylcarboxyle, alcoxy ou phénoxy ; R² représente un atome d'hydrogène ou un groupe alkyle ou phényle ; R³ est choisi parmi les groupes de formules 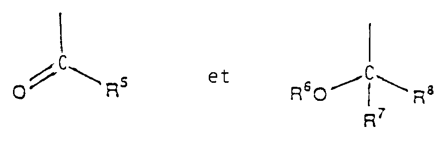 dans lesquelles R⁵, R⁶, R⁷ et R⁸ représentent chacun indépendamment un atome d'hydrogène ou un groupe alkyle, cycloalkyle, phényle ou phénylalkyle ; R⁴ représente un atome d'hydrogène ou un groupe alkyle, cyano, cycloalkyle, cycloalkylalkyle, cycloalkylcarbonyle, allyle, phényle, benzyle, phénéthyle ou phénylpropyle ; et dans laquelle l'un quelconque des substituants R¹, R², R³, R⁴, R⁵, R⁶, R⁷ et R⁸ précédents ayant une position substituable peut être substitué par un ou plusieurs substituants choisis parmi les halogènes et les groupes hydroxyle, alkyle et cycloalkyle.

5. Composé selon la revendication 4, dans lequel R¹ représente un groupe hydroxyle, alkylcarboxyle, alcoxy ou phénoxy ; R² représente un atome d'hydrogène ou un groupe alkyle ou phényle ; R⁵, R⁶, R⁷ et R⁸ représentent chacun indépendamment un atome d'hydrogène ou un groupe alkyle ou cycloalkyle ; et R⁴ représente un atome d'hydrogène ou un groupe cyano, alkyle, cycloalkyle, cycloalkylalkyle, cycloalkylcarbonyle ou allyle.

6. Composé selon la revendication 5, dans lequel R¹ est choisi parmi les groupes hydroxyle, méthylcarboxyle, méthoxy, éthoxy, n-propoxy, isopropoxy, n-butoxy, isobutoxy, sec-butoxy, tert-butoxy et phénoxy ; R² est choisi parmi un atome d'hydrogène et les groupes méthyle, éthyle, n-propyle, isopropyle, n-butyle, isobutyle, sec-butyle, tert-butyle, acétyle et phényle ; R⁵, R⁶, R⁷ et R⁸ représentent chacun indépendamment un atome d'hydrogène ou un groupe méthyle, éthyle, n-propyle, isopropyle, n-butyle, isobutyle, sec-butyle ou tert-butyle ; et R⁴ est choisi parmi un atome d'hydrogène et les groupes cyano, méthyle, éthyle, n-propyle, isopropyle, n-butyle, isobutyle, sec-butyle, tert-butyle, cyclopropyle, méthyle, allyle et cyclopropylcarbonyle.

7. Composé selon la revendication 6, dans lequel R⁵, R⁷ et R⁸ représentent chacun un groupe méthyle, éthyle, n-propyle, isopropyle, n-butyle, isobutyle, sec-butyle ou tert-butyle ; et R⁶ est un atome d'hydrogène.

8. Composé selon la revendication 7, dans lequel R³ est choisi parmi les groupes de formules

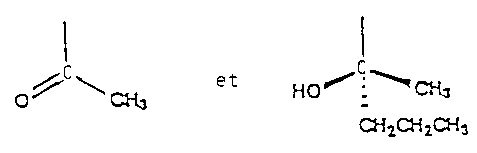

9. Composé selon la revendication 8, qui a une double liaison entre les atomes de carbone en positions 17 et 18 du pont carbone-carbone reliant les atomes de carbone en positions 6 et 14.

10. Composé selon la revendication 9, qui est l'énantiomère (+) de la 7α-(1-(R)-hydroxy-1-méthylbutyl)-6,14-endo-éthénotétrahydrooripavine.

11. Composé selon la revendication 9, qui est l'énantiomère (+) de la 7α-(1-(R)-hydroxy-1-méthylbutyl)-6,14-endo-éthénotétrahydrothébaïne.

12. Composé selon la revendication 9, qui est l'énantiomère (+) de la 7α-acétyl-6,14-endo-éthénotétrahydrothébaïne.

13. Composé selon la revendication 8 qui a une liaison simple entre les atomes de carbone en positions 17 et 18 du pont carbone-carbone reliant les atomes de carbone en positions 6 et 14.

14. Composé selon la revendication 13, qui est choisi parmi les suivants : énantiomère (+) de la 7α-acétyl-6,14-endo-éthanotétrahydrothébaïne ; énantiomère (+) de la 7α-(1-hydroxy-1-méthyléthyl)-6,14-endo-éthanotétrahydrothébaïne ; énantiomère (+) de la N-cyano-7a-(1-hydroxy-1-méthyléthyl)-6,14-endo-éthanotétrahydronorthébaïne ; énantiomère (+) de la 7α-(1-hydroxy-1-méthyléthyl)-6,14-endo-éthanotétrahydronororipavine ; énantiomère (+) de la N-cyclopropylcarbonyl-7α-(1-hydroxy-1-méthyléthyl)-6,14-endo-éthanotétrahydronororipavine ; et énantiomère (+) de la N-cyclopropylméthyl-7α-(1-hydroxy-1-méthyléthyl)-6,14-endo-éthanotétrahydronororipavine.

15. Composé selon la revendication 14 qui est l'énantiomère (+) de la N-cyclopropylméthyl-7α-(1-(R)-hydroxy-1-méthyléthyl)-6,14-endo-éthanotétrahydronororipavine.

16. Composé selon la revendication 13, choisi parmi les suivants : énantiomère (+) de la 7α-(1-hydroxy-1-tert-butyléthyl)-6,14-endo-éthanotétrahydrothébaïne ; énantiomère (+) de la N-cyano-7α-(1-hydroxy-1-tert-butyléthyl)-6,14-endo-éthanotétrahydronorthébaïne ; et énantiomère (+) de la N-cyclopropylméthyl-7α-(1-hydroxy-1-tert-butyléthyl)-6,14-endo-éthanotétrahydronororipavine.

17. Composé selon la revendication 16, qui est l'énantiomère (+) de la N-cyclopropylméthyl-7α-(1-(R)-hydroxy-1-tert-butyléthyl)-6,14-endo-éthanotétrahydronororipavine.

18. Composition pharmaceutique comprenant un dérivé d'époxymorphinane pratiquement non opiacé et un ou plusieurs excipients acceptables en pharmacie, ledit dérivé d'époxymorphinane non opiacé étant constitué par un ou plusieurs composés répondant à la formule selon la revendication 1.

19. Composition pharmaceutique selon la revendication 18, dans laquelle le dérivé d'époxymorphinane non opiacé est choisi parmi un groupe de composés selon l'une quelconque des revendications 2 à 17.

20. Utilisation d'un composé selon la revendication 1 pour préparer un médicament pour supprimer la toux chez un sujet sensible à la toux ou affligé de toux.

21. Utilisation selon la revendication 20, dans laquelle le composé est choisi parmi un groupe de composés selon l'une quelconque des revendications 2 à 17.

## Revendications (Revendications pour l'(les) Etat(s) contractant(s) suivant(s): ES)

1. Procédé pour préparer un composé de formule : 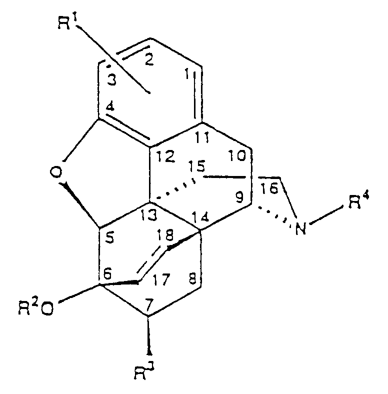 dans laquelle R¹ représente un atome d'hydrogène ou un groupe hydroxyle, alkyle, hydroxyalkyle, halogénoalkyle, cycloalkyle, cycloalkylalkyle, alcoxyalkyle, alcoxy, alcoxycarbonyle, alkylcarboxyle, aryle, aralkyle, aryloxy, arylthio, alkylthio, amino, amido ou carboxyle ; R² représente un atome d'hydrogène ou un groupe alkyle, hydroxyalkyle, halogénoalkyle, alcoxyalkyle, halogénoacyle, aryle ou aralkyle ; R³ représente un groupe alkyle, hydroxyalkyle, halogénoalkyle, cycloalkyle, cycloalkylalkyle, alcoxyalkyle, alcénylalkyle, alcoxy, aryle, aralkyle, aryloxy, alkylthio, amino, amido, carboxyle ou un groupe répondant à l'une des formules 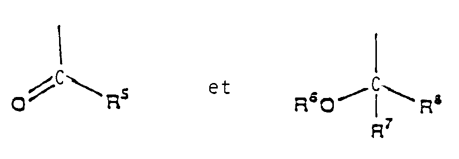 dans lesquelles R⁵, R⁶, R⁷ et R⁸ représentent chacun indépendamment un atome d'hydrogène ou un groupe alkyle, cycloalkyle, phényle ou phénylalkyle ; R⁴ représente un atome d'hydrogène ou un groupe alkyle, hydroxyalkyle, halogénoalkyle, cyano, cycloalkyle, cycloalkylalkyle, cycloalkylcarbonyle, alcénylalkyle, alcoxyalkyle, aryle, aralkyle ou carboxyle ; dans laquelle l'un quelconque des substituants R¹, R², R³ et R⁴ précédents ayant une position substituable peut être substitué par un ou plusieurs substituants choisis parmi les halogènes et les groupes hydroxyle, alkyle, cycloalkyle, aryle et aralkyle ; et dans laquelle le trait interrompu entre les atomes de carbone en positions 17 et 18 du pont carbone-carbone reliant les atomes de carbone en positions 6 et 14 représente une liaison simple ou une liaison double ; ou un de ses sels acceptables en pharmacie, caractérisé en ce qu'un composé de formule (a) 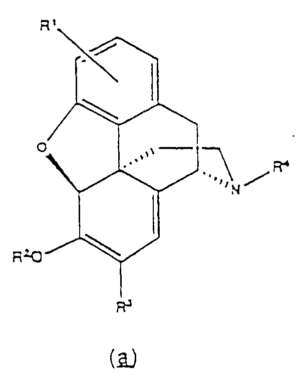 dans laquelle les restes R¹ à R⁴ sont tels que définis ci-dessus est mis à réagir dans des conditions appropriées avec une vinylcétone de formule (b) 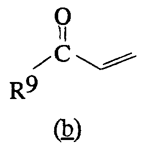 dans laquelle R⁹ est un reste précurseur à introduire dans le substituant R³ du produit final,
conduisant ainsi au composé de formule (c) 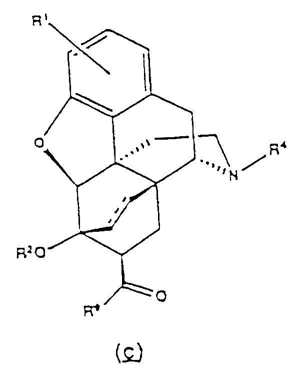 dans laquelle tous les substituants sont tels que définis ci-dessus, ledit composé (c) étant ensuite mis à réagir avec un réactif de Grignard de formule (d) 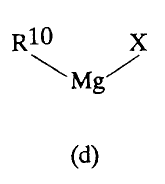 dans laquelle X est un atome d'halogène et R¹⁰ est un reste précurseur à introduire dans le substituant R³ du produit final, conduisant ainsi aux composés finaux désirés, qui peuvent facultativement être transformés de manière connue en leurs sels respectifs acceptables en pharmacie.

2. Procédé selon la revendication 1, dans lequel R¹ dans le composé préparé représente un atome d'hydrogène ou un groupe hydroxyle, alkyle, hydroxyalkyle, alcoxyalkyle, alcoxy, alcoxycarbonyle, alkylcarboxyle, phénoxy ou phénylthio ; R² représente un atome d'hydrogène ou un groupe alkyle, hydroxyalkyle, alcoxyalkyle, phényle ou phénylalkyle ; R³ représente un groupe alkyle, hydroxyalkyle, halogénoalkyle, cycloalkyle, cycloalkylalkyle, cycloalkylcarbonyle, alcénylcarbonyle, alcoxyalkyle ou un groupe répondant à l'une des formules 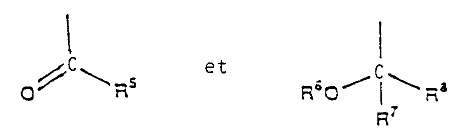 dans lesquelles R⁵, R⁶, R⁷ et R⁸ représentent chacun indépendamment un atome d'hydrogène ou un groupe alkyle, cycloalkyle, phényle ou phénylalkyle ; R⁴ représente un atome d'hydrogène ou un groupe alkyle, hydroxyalkyle, halogénoalkyle, cyano, cycloalkyle, cycloalkylalkyle, cycloalkylcarbonyle, alcénylalkyle, alcoxyalkyle, phényle ou phénylalkyle ; et dans laquelle l'un quelconque des substituants R¹, R², R³ et R⁴ précédents ayant une position substituable peut être substitué par un ou plusieurs substituants choisis parmi les halogènes et les groupes hydroxyle, alkyle, cycloalkyle, phényle et phénylalkyle.

3. Procédé selon la revendication 2, dans lequel R¹ dans le composé préparé représente un groupe hydroxyle, alkyle, hydroxyalkyle, alcoxyalkyle, alcoxy, alkylcarboxyle ou phénoxy ; R² représente un atome d'hydrogène ou un groupe alkyle, hydroxyalkyle, alcoxyalkyle, phényle ou phénylalkyle ; R³ représente un groupe alkyle, hydroxyalkyle, halogénoalkyle, cycloalkyle, cycloalkylalkyle, alcoxyalkyle ou un groupe répondant à l'une des formules 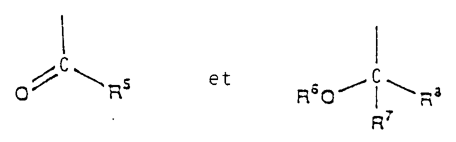 dans lesquelles R⁵, R⁶, R⁷ et R⁸ représentent chacun indépendamment un atome d'hydrogène ou un groupe alkyle, cycloalkyle, phényle ou phénylalkyle ; R⁴ représente un atome d'hydrogène ou un groupe alkyle, cyano, cycloalkyle, cycloalkylalkyle, cycloalkylcarbonyle, allyle, alcoxyalkyle, phényle ou phénylalkyle ; et dans laquelle l'un quelconque des substituants R¹, R², R³ et R⁴ précédents ayant une position substituable peut être substitué par un ou plusieurs substituants choisis parmi les halogènes et les groupes hydroxyle, alkyle, cycloalkyle, phényle et phénylalkyle.

4. Procédé selon la revendication 3, dans lequel R¹ dans le composé préparé représente un groupe hydroxyle, alkylcarboxyle, alcoxy ou phénoxy ; R² représente un atome d'hydrogène ou un groupe alkyle ou phényle ; R³ est choisi parmi les groupes de formules 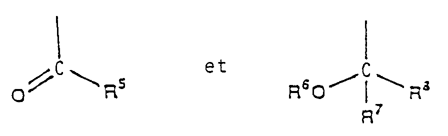 dans lesquelles R⁵, R⁶, R⁷ et R⁸ représentent chacun indépendamment un atome d'hydrogène ou un groupe alkyle, cycloalkyle, phényle ou phénylalkyle ; R⁴ représente un atome d'hydrogène ou un groupe alkyle, cyano, cycloalkyle, cycloalkylalkyle, cycloalkylcarbonyle, allyle, phényle, benzyle, phénéthyle ou phénylpropyle ; et dans laquelle l'un quelconque des substituants R¹, R², R³, R⁴, R⁵, R⁶, R⁷ et R⁸ precédents ayant une position substituable peut être substitué par un ou plusieurs substituants choisis parmi les halogènes et les groupes hydroxyle, alkyle et cycloalkyle.

5. Procédé selon la revendication 4, dans lequel R¹ dans le composé préparé représente un groupe hydroxyle, alkylcarboxyle, alcoxy ou phénoxy ; R² représente un atome d'hydrogène ou un groupe alkyle ou phényle ; R⁵, R⁶, R⁷ et R⁸ représentent chacun indépendamment un atome d'hydrogène ou un groupe alkyle ou cycloalkyle ; et R⁴ représente un atome d'hydrogène ou un groupe cyano, alkyle, cycloalkyle, cycloalkylalkyle, cycloalkylcarbonyle ou allyle.

6. Procédé selon la revendication 4, dans lequel R¹ dans le composé préparé est choisi parmi les groupes hydroxyle, méthylcarboxyle, méthoxy, éthoxy, n-propoxy, isopropoxy, n-butoxy, isobutoxy, sec-butoxy, tert-butoxy et phénoxy; R² est choisi parmi un atome d'hydrogène et les groupes méthyle, éthyle, n-propyle, isopropyle, n-butyle, isobutyle, sec-butyle, tert-butyle, acétyle et phényle ; R⁵, R⁶, R⁷ et R⁸ représentent chacun indépendamment un atome d'hydrogène ou un groupe méthyle, éthyle, n-propyle, isopropyle, n-butyle, isobutyle, sec-butyle ou tert-butyle ; et R⁴ est choisi parmi un atome d'hydrogène et les groupes cyano, méthyle, éthyle, n-propyle, isopropyle, n-butyle, isobutyle, sec-butyle, tert-butyle, cyclopropyle, méthyle, allyle et cyclopropylcarbonyle.

7. Procédé selon la revendication 6, dans lequel R⁵, R⁷ et R⁸ représentent chacun un groupe méthyle, éthyle, n-propyle, isopropyle, n-butyle, isobutyle, sec-butyle ou tert-butyle; et R⁶ est un atome d'hydrogène.

8. Procédé selon la revendication 7, dans lequel R³ est choisi parmi les groupes de formules

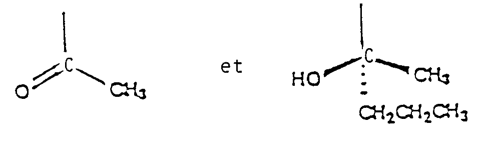

9. Procédé selon la revendication 8, dans lequel le composé préparé a une double liaison entre les atomes de carbone en positions 17 et 18 du pont carbone-carbone reliant les atomes en positions 6 et 14.

10. Procédé selon la revendication 9, dans lequel le composé préparé est l'énantiomère (+) de la 7α-(1-(R)-hydroxy-1-méthylbutyl)-6,14-endo-éthénotétrahydrooripavine.

11. Procédé selon la revendication 9, qui est l'énantiomère (+) de la 7α-(1-(R)-hydroxy-1-méthylbutyl)-6,14-endo-éthénotétrahydrothébaïne.

12. Procédé selon la revendication 9, qui est l'énantiomère (+) de la 7α-acétyl-6,14-endo-éthénotétrahydrothébaïne.

13. Procédé selon la revendication 8, dans lequel le composé préparé a une liaison simple entre les atomes de carbone en positions 17 et 18 du pont carbone-carbone reliant les atomes de carbone en positions 6 et 14.

14. Procédé selon la revendication 13, dans lequel le composé préparé est choisi parmi les suivants : énantiomère (+) de la 7α-acétyl-6,14-endo-éthanotétrahydrothébaïne ; énantiomère (+) de la 7α-(1-hydroxy-1-méthyléthyl)-6,14-endo-éthanotétrahydrothébaïne ; énantiomère (+) de la N-cyano-7α-(1-hydroxy-1-méthyléthyl)-6,14-endo-éthanotétrahydronorthébaïne; énantiomère (+) de la 7α-(1-hydroxy-1-méthyléthyl)-6,14-endo-éthanotétrahydronororipavine ; énantiomère (+) de la N-cyclopropylcarbonyl-7α-(1-hydroxy-1-méthyléthyl)-6,14-endo-éthanotétrahydronororipavine ; et énantiomère (+) de la N-cyclopropylméthyl-7α-(1-hydroxy-1-méthyléthyl)-6,14-endo-éthanotétrahydronororipavine.

15. Procédé selon la revendication 14, dans lequel le composé préparé est l'énantiomère (+) de la N-cyclopropylméthyl-7α-(1-(R)-hydroxy-1-méthyléthyl)-6,14-endo-éthanotétrahydronororipavine.

16. Procédé selon la revendication 13, dans lequel le composé préparé est choisi parmi les suivants : énantiomère (+) de la 7α-(1-hydroxy-1-tert-butyléthyl)-6,14-endo-éthanotétrahydrothébaïne ; énantiomère (+) de la N-cyano-7α-(1-hydroxy-1-tert-butyléthyl)-6,14-endoéthanotétrahydronorthébaïne ; et énantiomère (+) de la N-cyclopropylméthyl-7α-(1-hydroxy-1-tert-butyléthyl)-6,14-endo-éthanotétrahydronororipavine.

17. Procédé selon la revendication 16, dans lequel le composé préparé est l'énantiomère (+) de la N-cyclopropylméthyl-7α-(1-(R)-hydroxy-1-tert-butyléthyl)-6,14-endo-éthanotétrahydronororipavine.

18. Procédé pour préparer une composition pharmaceutique consistant à mélanges un ou plusieurs supports acceptables en pharmacie avec un dérivé d'époxymorphinane pratiquement non opiacé et un ou plusieurs excipients acceptables en pharmacie, caractérisé en ce que ledit dérivé d'époxymorphinane non opiacé est constitué par un ou plusieurs composés de formule ci-dessus tels que préparés selon la revendication 1.

19. Procédé selon la revendication 18, caractérisé en ce que le dérivé d'époxymorphinane est choisi parmi un groupe de composés tels que préparés selon l'une quelconque des revendications 2 à 17.

20. Utilisation d'un composé tel que préparé selon la revendication 1 pour préparer un médicament pour supprimer la toux chez un sujet sensible à la toux ou affligé de toux.

21. Utilisation selon la revendication 20, dans laquelle le composé est choisi parmi un groupe de composés tels que préparés selon l'une quelconque des revendications 2 à 17.

## Revendications (Revendications pour l'(les) Etat(s) contractant(s) suivant(s): GR)

1. Composé de formule : 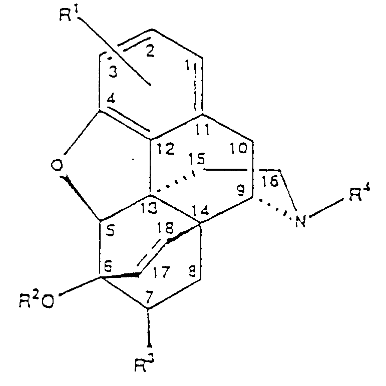 dans laquelle R¹ représente un atome d'hydrogène ou un groupe hydroxyle, alkyle, hydroxyalkyle, halogénoalkyle, cycloalkyle, cycloalkylalkyle, alcoxyalkyle, alcoxy, alcoxycarbonyle, alkylcarboxyle, aryle, aralkyle, aryloxy, arylthio, alkylthio, amino, amido ou carboxyle ; R² représente un atome d'hydrogène ou un groupe alkyle, hydroxyalkyle, halogénoalkyle, alcoxyalkyle, halogénoacyle, aryle ou aralkyle ; R³ représente un groupe alkyle, hydroxyalkyle, halogénoalkyle, cycloalkyle, cycloalkylalkyle, alcoxyalkyle, alcénylalkyle, alcoxy, aryle, aralkyle, aryloxy, alkylthio, amino, amido, carboxyle ou un groupe répondant à l'une des formules 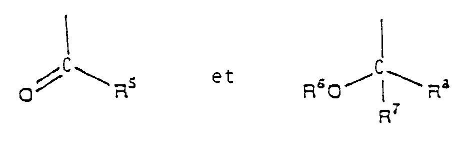 dans lesquelles R⁵, R⁶, R⁷ et R⁸ représentent chacun indépendamment un atome d'hydrogène ou un groupe alkyle, cycloalkyle, phényle ou phénylalkyle ; R⁴ représente un atome d'hydrogène ou un groupe alkyle, hydroxyalkyle, halogénoalkyle, cyano, cycloalkyle, cycloalkylalkyle, cycloalkylcarbonyle, alcénylalkyle, alcoxyalkyle, aryle, aralkyle ou carboxyle ; dans laquelle l'un quelconque des substituants R¹, R², R³ et R⁴ précédents ayant une position substituable peut être substitué par un ou plusieurs substituants choisis parmi les halogènes et les groupes hydroxyle, alkyle, cycloalkyle, aryle et aralkyle ; et dans laquelle le trait interrompu entre les atomes de carbone en positions 17 et 18 du pont carbone-carbone reliant les atomes de carbone en positions 6 et 14 représente une liaison simple ou une liaison double ; ou un de ses sels acceptables en pharmacie.

2. Composé selon la revendication 1, dans lequel R¹ représente un atome d'hydrogène ou un groupe hydroxyle, alkyle, hydroxyalkyle, alcoxyalkyle, alcoxy, alcoxycarbonyle, alkylcarboxyle, phénoxy ou phénylthio ; R² représente un atome d'hydrogène ou un groupe alkyle, hydroxyalkyle, alcoxyalkyle, phényle ou phénylalkyle; R³ représente un groupe alkyle, hydroxyalkyle, halogénoalkyle, cycloalkyle, cycloalkylalkyle, cycloalkylcarbonyle, alcénylcarbonyle, alcoxyalkyle ou un groupe répondant à l'une des formules 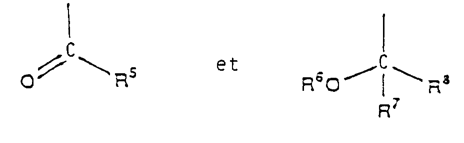 dans lesquelles R⁵, R⁶, R⁷ et R⁸ représentent chacun indépendamment un atome d'hydrogène ou un groupe alkyle, cycloalkyle, phényle ou phénylalkyle ; R⁴ représente un atome d'hydrogène ou un groupe alkyle, hydroxyalkyle, halogénoalkyle, cyano, cycloalkyle, cycloalkylalkyle, cycloalkylcarbonyle, alcénylalkyle, alcoxyalkyle, phényle ou phénylalkyle ; et dans laquelle l'un quelconque des substituants R¹, R², R³ et R⁴ précédents ayant une position substituable peut être substitué par un ou plusieurs substituants choisis parmi les halogènes et les groupes hydroxyle, alkyle, cycloalkyle, phényle et phénylalkyle.

3. Composé selon la revendication 2, dans lequel R¹ représente un groupe hydroxyle, alkyle, hydroxyalkyle, alcoxyalkyle, alcoxy, alkylcarboxyle ou phénoxy ; R² représente un atome d'hydrogène ou un groupe alkyle, hydroxyalkyle, alcoxyalkyle, phényle ou phénylalkyle ; R³ représente un groupe alkyle, hydroxyalkyle, halogénoalkyle, cycloalkyle, cycloalkylalkyle, alcoxyalkyle ou un groupe répondant à l'une des formules 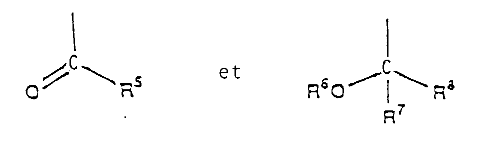 dans lesquelles R⁵, R⁶, R⁷ et R⁸ représentent chacun indépendamment un atome d'hydrogène ou un groupe alkyle, cycloalkyle, phényle ou phénylalkyle ; R⁴ représente un atome d'hydrogène ou un groupe alkyle, cyano, cycloalkyle, cycloalkylalkyle, cycloalkylcarbonyle, allyle, alcoxyalkyle, phényle ou phénylalkyle ; et dans laquelle l'un quelconque des substituants R¹, R², R³ et R⁴ précédents ayant une position substituable peut être substitué par un ou plusieurs substituants choisis parmi les halogènes et les groupes hydroxyle, alkyle, cycloalkyle, phényle et phénylalkyle.

4. Composé selon la revendication 3, dans lequel R¹ représente un groupe hydroxyle, alkylcarboxyle, alcoxy ou phénoxy ; R² représente un atome d'hydrogène ou un groupe alkyle ou phényle; R³ est choisi parmi les groupes de formules 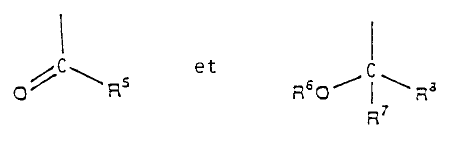 dans lesquelles R⁵, R⁶, R⁷ et R⁸ représentent chacun indépendamment un atome d'hydrogène ou un groupe alkyle, cycloalkyle, phényle ou phénylalkyle ; R⁴ représente un atome d'hydrogène ou un groupe alkyle, cyano, cycloalkyle, cycloalkylalkyle, cycloalkylcarbonyle, allyle, phényle, benzyle, phénéthyle ou phénylpropyle ; et dans laquelle l'un quelconque des substituants R¹, R², R³, R⁴, R⁵, R⁶, R⁷ et R⁸ précédents ayant une position substituable peut être substitué par un ou plusieurs substituants choisis parmi les halogènes et les groupes hydroxyle, alkyle et cycloalkyle.

5. Composé selon la revendication 4, dans lequel R¹ représente un groupe hydroxyle, alkylcarboxyle, alcoxy ou phénoxy ; R² représente un atome d'hydrogène ou un groupe alkyle ou phényle ; R⁵, R⁶, R⁷ et R⁸ représentent chacun indépendamment un atome d'hydrogène ou un groupe alkyle ou cycloalkyle; et R⁴ représente un atome d'hydrogène ou un groupe cyano, alkyle, cycloalkyle, cycloalkylalkyle, cycloalkylcarbonyle ou allyle.

6. Composé selon la revendication 5, dans laquelle R¹ est choisi parmi les groupes hydroxyle, méthylcarboxyle, méthoxy, éthoxy, n-propoxy, isopropoxy, n-butoxy, isobutoxy, sec-butoxy, tert-butoxy et phénoxy ; R² est choisi parmi un atome d'hydrogène et les groupes méthyle, éthyle, n-propyle, isopropyle, n-butyle, isobutyle, sec-butyle, tert-butyle, acétyle et phényle ; R⁵, R⁶, R⁷ et R⁸ représentent chacun indépendamment un atome d'hydrogène ou un groupe méthyle, éthyle, n-propyle, isopropyle, n-butyle, isobutyle, sec-butyle ou tert-butyle ; et R⁴ est choisi parmi un atome d'hydrogène et les groupes cyano, méthyle, éthyle, n-propyle, isopropyle, n-butyle, isobutyle, sec-butyle, tert-butyle, cyclopropyle, méthyle, allyle et cyclopropylcarbonyle.

7. Composé selon la revendication 6, dans lequel R⁵, R⁷ et R⁸ représentent chacun un groupe méthyle, éthyle, n-propyle, isopropyle, n-butyle, isobutyle, sec-butyle ou tert-butyle ; et R⁶ est un atome d'hydrogène.

8. Composé selon la revendication 7, dans lequel R³ est choisi parmi les groupes de formules

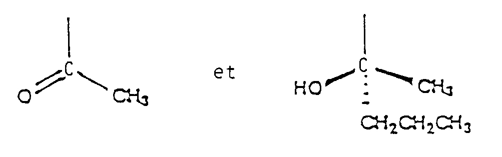

9. Composé selon la revendication 8, qui a une double liaison entre les atomes de carbone en positions 17 et 18 du pont carbone-carbone reliant les atomes de carbone en positions 6 et 14.

10. Composé selon la revendication 9, qui est l'énantiomère (+) de la 7α-(1-(R)-hydroxy-1-méthylbutyl)-6,14-endo-éthénotétrahydrooripavine.

11. Composé selon la revendication 9, qui est l'énantiomère (+) de la 7α-(1-(R)-hydroxy-1-méthylbutyl)-6,14-endo-éthénotétrahydrothébaïne.

12. Composé selon la revendication 9, qui est l'énantiomère (+) de la 7α-acétyl-6,14-endo-éthénotétrahydrothébaïne.

13. Composé selon la revendication 8 qui a une liaison simple entre les atomes de carbone en positions 17 et 18 du pont carbone-carbone reliant les atomes de carbone en positions 6 et 14.

14. Composé selon la revendication 13, qui est choisi parmi les suivants : énantiomère (+) de la 7α-acétyl-6,14-endo-éthanotétrahydrothébaïne ; énantiomère (+) de la 7α-(1-hydroxy-1-méthyléthyl)-6,14-endo-éthanotétrahydrothébaïne ; énantiomère (+) de la N-cyano-7α-(1-hydroxy-1-méthyléthyl)-6,14-endo-éthanotétrahydronorthébaïne ; énantiomère (+) de la 7α-(1-hydroxy1-méthyléthyl)-6,14-endo-éthanotétrahydronororipavine ; énantiomère (+) de la N-cyclopropylcarbonyl-7α-(1-hydroxy-1-méthyléthyl)-6,14-endo-éthanotétrahydronororipavine ; et énantiomère (+) de la N-cyclopropylméthyl-7α-(1-hydroxy-1-méthyléthyl)-6,14-endo-éthanotétrahydronororipavine.

15. Composé selon la revendication 14 qui est l'énantiomère (+) de la N-cyclopropylméthyl-7α-(1-(R)-hydroxy-1-méthyléthyl)-6,14-endo-éthanotétrahydronororipavine.

16. Composé selon la revendication 13, choisi parmi les suivants : énantiomère (+) de la 7α-(1-hydroxy-1-tert-butyléthyl)-6,14-endo-éthanotétrahydrothébaïne ; énantiomère (+) de la N-cyano-7α-(1-hydroxy-1-tert-butyléthyl)-6,14-endo-éthanotétrahydronorthébaïne ; et énantiomère (+) de la N-cyclopropylméthyl-7α-(1-hydroxy-1-tert-butyléthyl)-6,14-endo-éthanotétrahydronororipavine.

17. Composé selon la revendication 16, qui est l'énantiomère (+) de la N-cyclopropylméthyl-7α-(1-(R)-hydroxy-1-tert-butyléthyl)-6,14-endo-éthanotétrahydronororipavine.

18. Procédé pour préparer une composition pharmaceutique consistant à mélanger un ou plusieurs supports acceptables en pharmacie avec un dérivé d'époxymorphinane pratiquement non opiacé et un ou plusieurs excipients acceptables en pharmacie, caractérisé en ce que ledit dérivé d'époxymorphinane non opiacé est constitué par un ou plusieurs composés de formule ci-dessus selon la revendication 1.

19. Procédé selon la revendication 18, caractérisé en ce que le dérivé d'époxymorphinane est choisi parmi un groupe de composés selon l'une quelconque des revendications 2 à 17.

20. Utilisation d'un composé selon la revendication 1 pour préparer un médicament pour supprimer la toux chez un sujet sensible à la toux ou affligé de toux.

21. Utilisation selon la revendication 20, dans laquelle le composé est choisi parmi un groupe de composés selon l'une quelconque des revendications 2 à 17.
